(19) Europäisches Patentamt / European Patent Office / Office européen des brevets

(11) **EP 4 541 678 A1**

(12) **EUROPEAN PATENT APPLICATION**
published in accordance with Art. 153(4) EPC

(43) Date of publication:
**23.04.2025 Bulletin 2025/17**

(21) Application number: **23823552.7**

(22) Date of filing: **02.05.2023**

(51) International Patent Classification (IPC):
**B60W 40/08** (2012.01)   **A61B 5/18** (2006.01)
**A61B 10/00** (2006.01)   **B60R 11/02** (2006.01)
**B60R 16/037** (2006.01)   **B60W 40/10** (2012.01)
**B60W 50/08** (2020.01)   **G08G 1/16** (2006.01)

(52) Cooperative Patent Classification (CPC):
**A61B 5/18; A61B 10/00; B60R 11/02;**
**B60R 16/037; B60W 40/08; B60W 40/10;**
**B60W 50/08; G08G 1/16**

(86) International application number:
**PCT/JP2023/017117**

(87) International publication number:
**WO 2023/243249 (21.12.2023 Gazette 2023/51)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **14.06.2022 JP 2022096131**

(71) Applicant: **Sony Group Corporation**
**Tokyo 108-0075 (JP)**

(72) Inventors:
 • **TAKAMATSU, Takashi**
   **Tokyo 108-0075 (JP)**
 • **WATANABE, Naoki**
   **Tokyo 108-0075 (JP)**
 • **IMAMURA, Hiroshi**
   **Tokyo 108-0075 (JP)**
 • **TOMONAGA, Seishi**
   **Tokyo 108-0075 (JP)**
 • **KUBOTA, Hiroyuki**
   **Tokyo 108-0075 (JP)**
 • **TAKEUCHI, Hiroshi**
   **Tokyo 108-0075 (JP)**

(74) Representative: **Witte, Weller & Partner**
**Patentanwälte mbB**
**Postfach 10 54 62**
**70047 Stuttgart (DE)**

(54) **INFORMATION PROCESSING DEVICE, INFORMATION PROCESSING METHOD, AND SYSTEM**

(57) To provide an apparatus that processes information regarding motion sickness of an occupant in a movable object.

The information processing apparatus includes: a motion sickness accumulation computing unit that calculates an accumulation factor of motion sickness of an occupant in a movable object; a motion sickness recovery computing unit that calculates a recovery factor of the motion sickness of the occupant; and a motion sickness computing unit that combines the accumulation factor and the recovery factor to calculate a motion sickness index of the occupant. The motion sickness accumulation computing unit computes the accumulation factor by using a head motion of the occupant which is predicted by head motion simulation computing based on acceleration of the movable object and body information of the occupant.

Information processing system 100

FIG.1

**Description**

Technical Field

A technology disclosed in this specification (hereinafter, referred to as "the present disclosure") relates to an information processing apparatus, an information processing method, and a system that process information regarding motion sickness of an occupant in a movable object.

Background Art

While in a vehicle such as a car or boat, an occupant may feel queasy and exhibit symptoms of motion sickness over the course of a boarding time, but it is difficult to determine whether the occupant is developing motion sickness. ISO 2631 clearly states that a determination index for motion sickness can be calculated from a behavior (acceleration) of the occupant's head. However, ISO 2631 only describes a calculation method based on time integration, and in this calculation method, motion sickness will always occur after a certain time no matter what the behavior is. In other words, the calculation method described in ISO 2631 makes a determination in consideration of even small movements that do not lead to motion sickness, which may cause inconvenience.

For example, a motion sickness estimation apparatus has been proposed. The motion sickness estimation apparatus detects a head behavior of an occupant as an occupant motion and detects at least one of a seating posture, a restraint state, a body pressure distribution, a height, a weight, or a gender of an occupant. Then, the motion sickness estimation apparatus predicts motion sickness on the basis of driving data of a predetermined time when a longer time than the predetermined time has elapsed by calculation according to a motion sickness dose value (MSDV) of ISO 2631 (see Patent Literature 1).

Moreover, a motion sickness prevention method has been proposed. The motion sickness prevention method predicts whether motion sickness can be caused when an occupant undergoes the motion of a movable object for a long time on the basis of 3-direction acceleration components of the movable object (see Patent Literature 2). This method uses a transfer function with band-pass characteristics that can approximate the relationship between the acceleration of motion and the degree to which this motion can cause motion sickness in the average person to make predictions, and also uses an integrator to determine the cumulative degree of motion sickness measured over a specific time period.

A method of measuring a train motion sickness evaluation index on the basis of a combination of left-right acceleration, roll angle, and roll angular velocity measured by onboard sensors has also been proposed (see Patent Literature 3). In this method, sampling is performed on the basis of a sampling cycle of 100 Hz, and sampled values are integrated for 30 minutes for measurement.

Citation List

Patent Literature

    Patent Literature 1: Japanese Patent Application Laid-open No. 2007-236644
    Patent Literature 2: Japanese Patent Application Laid-open No. 2005-517568
    Patent Literature 3: Japanese Patent Application Laid-open No. 2004-286502

Disclosure of Invention

Technical Problem

It is an objective of the present disclosure to provide an information processing apparatus and an information processing method for calculating a motion sickness index of an occupant in a movable object and a system for controlling the operation of the movable object or the like on the basis of the calculated motion sickness index.

Solution to Problem

The present disclosure has been made in view of the above-mentioned problem and a first aspect thereof is an information processing apparatus including:

    a motion sickness accumulation computing unit that calculates an accumulation factor of motion sickness of an occupant in a movable object;
a motion sickness recovery computing unit that calculates a recovery factor of the motion sickness of the occupant; and
a motion sickness computing unit that combines the accumulation factor and the recovery factor to calculate a motion sickness index of the occupant.

The motion sickness accumulation computing unit computes the accumulation factor by using a head motion of the occupant which is predicted by head motion simulation computing based on acceleration of the movable object and body information of the occupant. Moreover, the motion sickness recovery factor computing unit computes the recovery factor on the basis of a traveling time of the movable object and body information of the occupant.

The motion sickness recovery factor computing unit may compute the recovery factor in consideration of at least one of a difference of a motion of the movable object according to the position of the occupant or a difference of field-of-view information according to the posture of the occupant. Moreover, the motion sickness recovery factor computing unit may compute the recovery factor also in consideration of how the movable object is traveling.

The information processing apparatus according to the first aspect may further include a processing unit that performs processing according to the motion sickness index of the occupant in the movable object. The processing unit may perform, for example, when the motion sickness of the occupant is predicted, at least one of information presentation to instruct limitation of at least one of a traveling speed or a route change in manual driving of the movable object, driving control associated with limitation of at least one of a traveling speed or a route change in automated driving of the movable object, or operation control of a display system mounted on the movable object.

Moreover, a second aspect of the present disclosure is an information processing method including:

a motion sickness accumulation computing step of calculating an accumulation factor of motion sickness of an occupant in a movable object;
a motion sickness recovery computing step of calculating a recovery factor of the motion sickness of the occupant; and
a motion sickness computing step of combining the accumulation factor and the recovery factor to calculate a motion sickness index of the occupant.

Moreover, a third aspect of the present disclosure is a system including:

a motion sickness accumulation computing unit that calculates an accumulation factor of motion sickness of an occupant in a movable object;
a motion sickness recovery computing unit that calculates a recovery factor of the motion sickness of the occupant; and
a motion sickness computing unit that combines the accumulation factor and the recovery factor to calculate a motion sickness index of the occupant;
a display system that is mounted on the movable object and displays a video to be viewed by the occupant; and
a processing unit that performs processing according to the motion sickness index of the occupant on the display system.

It should be noted that the "system" set forth therein refers to a logical set of a plurality of apparatuses (or functional modules that realize particular functions), and whether or not the respective apparatuses or the functional modules are in a single casing is not particularly relevant. That is, both a single apparatus consisting of a plurality of components or functional modules and a set of a plurality of apparatuses are equivalent to the "system".

Advantageous Effects of Invention

In accordance with the present disclosure, an information processing apparatus and an information processing method for calculating a motion sickness index of an occupant in a movable object in consideration of a recovery factor of motion sickness and a system for controlling the operation of the movable object or the like on the basis of the calculated motion sickness index can be provided.

It should be noted that the effects described herein are examples only, and effects provided by the present disclosure are not limited thereto. Moreover, the present disclosure may produce additional effects in addition to the above-mentioned effects.

Other objectives, features, and advantages of the present disclosure will become apparent from more detailed descriptions based on embodiments to be described later and the accompanying drawings.

Brief Description of Drawings

[Fig. 1] Fig. 1 is a diagram showing a functional configuration of an information processing system 100 that estimates motion sickness of an occupant in a vehicle.

[Fig. 2] Fig. 2 is a flowchart showing a general operation procedure performed on the information processing system 100.

[Fig. 3] Fig. 3 is a diagram showing an internal configuration example of a head motion computing unit 101.

[Fig. 4] Fig. 4 is a flowchart showing a processing procedure for calculating a head motion of the occupant in the head motion computing unit 101 shown in Fig. 3.

[Fig. 5] Fig. 5 is a diagram showing an internal configuration example of a motion sickness accumulation factor computing unit 102.

[Fig. 6] Fig. 6 is a flowchart showing a processing procedure for calculating an accumulation factor of motion sickness of the occupant in the motion sickness accumulation factor computing unit 102 shown in Fig. 5.

[Fig. 7] Fig. 7 is a diagram showing an internal configuration example of a motion sickness recovery factor computing unit 103.

[Fig. 8] Fig. 8 is a flowchart showing a processing procedure for calculating the accumulation factor of motion sickness of the occupant in the motion sickness recovery factor computing unit 103 shown in Fig. 7.

[Fig. 9] Fig. 9 is a diagram extracting and showing a motion sickness computing unit 104 and an input/output part thereof.

[Fig. 10] Fig. 10 is a flowchart showing a processing procedure for calculating the motion sickness of the occupant in the motion sickness computing unit 104 shown in Fig. 9.

[Fig. 11] Fig. 11 is a diagram showing a functional configuration of an information processing system 1100 that estimates the motion sickness of the occupant in the vehicle.

[Fig. 12] Fig. 12 is a flowchart showing an operation procedure performed in the information processing system 1100.

[Fig. 13] Fig. 13 is a diagram showing a filter transfer function H(s) that converts vehicle acceleration a(t) into weighted effective acceleration $a_w(t)$.

[Fig. 14] Fig. 14 is a diagram showing experimental results of an MSDV and an SSQ according to how the vehicle is traveling.

[Fig. 15] Fig. 15 is a diagram showing prediction of the MSDV and the SSQ according to how the vehicle is traveling, superimposed on experimental results.

[Fig. 16] Fig. 16 is a diagram schematically showing the correlation between the MSDV and the SSQ in each case of acceleration and deceleration driving and normal driving.

[Fig. 17] Fig. 17 is a diagram showing a distribution of the MSDV and the SSQ during the driving to avoid the motion sickness.

[Fig. 18] Fig. 18 is a diagram showing an example of experimental results of the MSDV and an SSQ total score when the vehicle performs the same driving.

[Fig. 19] Fig. 19 is a view illustrating an overhead view of a cabin of a 3-row seat car.

[Fig. 20] Fig. 20 is a view showing a state in which a content video is displayed by a display system mounted on the vehicle.

[Fig. 21] Fig. 21 is a view showing a state in which the content video is displayed by the display system mounted on the vehicle (an example in which the transmittance is adjusted).

[Fig. 22] Fig. 22 is a view showing a state in which the content video is displayed by the display system mounted on the vehicle (an example in which the transmittance is adjusted).

[Fig. 23] Fig. 23 is a view showing a variation of a arrangement position of a transmittance-variable region in a vehicle-mounted display system.

[Fig. 24] Fig. 24 is a view showing a projector arrangement position corresponding to a transparent screen for RSE.

[Fig. 25] Fig. 25 is a view showing a projector arrangement position corresponding to a transparent screen arranged on a front window.

[Fig. 26] Fig. 26 is a view showing a projector arrangement position corresponding to a transparent screen arranged on a rear window.

[Fig. 27] Fig. 27 is a view showing a projector arrangement position corresponding to a transparent screen arranged on a side window.

[Fig. 28] Fig. 28 is a view showing an operation example of the display system for reducing the motion sickness (making the video transparent).

[Fig. 29] Fig. 29 is a view showing an operation example of the display system for reducing the motion sickness (making the video transparent and rotating the video).

[Fig. 30] Fig. 30 is a view showing an operation example of the display system for reducing the motion sickness (making the video transparent and moving the video).
[Fig. 31] Fig. 31 is a view showing an operation example of the display system for reducing the motion sickness (making the video transparent and reducing the size of the video).
[Fig. 32] Fig. 32 is a view showing an operation example of the display system for reducing the motion sickness (making the video transparent, rotating the video, and moving the video).
[Fig. 33] Fig. 33 is a view showing an operation example of the display system for reducing the motion sickness (making the video transparent, rotating the video, and reducing the size of the video).
[Fig. 34] Fig. 34 is a view showing an operation example of the display system for reducing the motion sickness (making the video transparent, moving the video, and reducing the size of the video).
[Fig. 35] Fig. 35 is a view showing an operation example of the display system for reducing the motion sickness (making the video transparent, rotating the video, moving the video, and reducing the size of the video).

Mode(s) for Carrying Out the Invention

Hereinafter, the present disclosure will be described in the following order with reference to the drawings.

A. Overview
B. Calculation Method for Motion Sickness Index
C. Recovery factor of Motion Sickness

C-1. Case of Shifting from City Driving to Highway Driving
C-2. In Case Where Vehicle is Automatically Driven at Very Low Speed
C-3. Changes in Motion Sickness Recovery Degree due to Orientations of Front Passenger Seat and Second-Row and Third-Row Seats
C-4. Changes in Motion Sickness Recovery Degree due to Content Display Control

D. System Configuration and Operation

D-1. Simulation Calculation of Head Motion of Occupant
D-2. Calculation for Accumulation factor of Motion Sickness
D-3. Calculation for Accumulation factor of Motion Sickness
D-4. Computing Operation for Motion Sickness
D-5. Changes in Motion Sickness Recovery Degree due to Orientations of Front Passenger Seat and Second-Row and Third-Row Seats

E. Display System and Control Thereof

E-1. Configuration of Display System
E-2. Reduction of Motion Sickness

F. Summary

A. Overview

While in a vehicle such as a car, motion sickness Symptoms occur due to influences of the car's behaviors. When motion sickness occurs, the occupant has difficulty continuing to board the vehicle and is greatly disturbed. The present disclosure relates to prediction of motion sickness in order to predict a motion sickness level of the occupant before motion sickness is developed and to link this to response processing such as vehicle motion control (e.g., driving instructions) and notification to the occupant or driver.
As described in the Background section, a method of calculating a motion sickness index by integrating the time of a head motion of the occupant has been used with regard to motion sickness (see, for example, Patent Literatures 1 and 3). However, it has been confirmed through actual experimental evaluation that even if the time-integrated value of the head motion is the same, the motion sickness Symptoms differ depending on how the boarded vehicle is traveling (i.e., there are cases where the motion sickness Symptoms are produced and cases where the motion sickness Symptoms are not produced). Based on this experimental evaluation, it can be considered that the occupant tends to recover from the motion sickness when there is a less head motion during a vehicle boarding time. The experimental formula can also generally describe that there is a tendency to recover from motion sickness. Therefore, in the present disclosure, a technology of calculating a motion sickness index by considering a recovery factor of motion sickness.

Moreover, in a case of predicting motion sickness on the basis of the head motion of the occupant, a method of directly detecting a head motion is difficult, and the accuracy of detection is also poor. Therefore, in view of the difficulty of directly detecting a head motion, the present disclosure also proposes a technology of calculating the head motion of the occupant by simulation on the basis of a vehicle motion. For example, a vehicle behavior can be accurately and easily detected on the basis of in-vehicle sensors such as acceleration sensors, and a head movement can be calculated by simulation prediction on the basis of physical information such as a weight and a sitting height of the occupant.

B. Calculation Method for Motion Sickness Index

ISO 2631 has described a calculation method for an MSDV as a determination index for motion sickness. According to the description, the determination index MSDV for the motion sickness is calculated using Expression (1) below.

[Formula 1]

$$MSDV = \sqrt{\int_0^T [a_W(t)]^2 dt} \qquad \cdots (1)$$

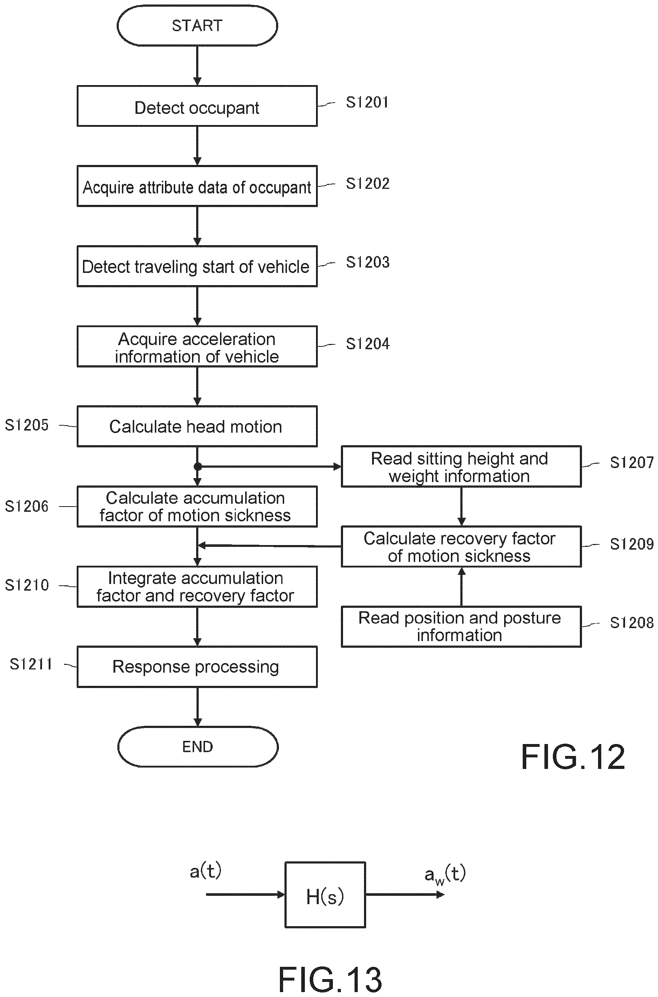

In Expression (1) above, $a_W(t)$ is weighted effective acceleration (weighted RMS acceleration) of the vehicle at a time t. The weighted effective acceleration $a_W(t)$ is acceleration obtained by weighting vehicle acceleration $a(t)$, which depends on a frequency. The weighted effective acceleration $a_W(t)$ can be obtained by converting the acceleration $a(t)$ of a time domain into a frequency domain temporarily, multiplying it by a transfer function $H(s)$ of the filter (It should be noted that $s = j\omega = j2\pi f$), and then converting it back to the time domain (see Fig. 13). It should be noted that the transfer function $H(s)$ of the filter depends on the motion (e.g., vehicle type). Therefore, in ISO 2631, the filter characteristics are changed by changing the coefficients $f_i$ (= $\omega_i$) and $Q_i$ for each case.

Moreover, as also mentioned above in Item A, in the present disclosure, in view of the fact that it is difficult to correctly detect the head motion of the occupant, the head motion of the occupant is calculated by simulation calculation on the basis of the motion of the vehicle. Specifically, a configuration in which a sensor such as an acceleration sensor is mounted on a rigid part of a vehicle body of the vehicle or the like is employed. Moreover, in order to detect attribute data of the occupant (body information such as a sitting height (h) and a weight (w) of an upper half of the body), a camera (e.g., in-vehicle monitoring camera) and a body pressure sensor are mounted on the vehicle. Then, head acceleration of the occupant which is calculated by simulation calculation is weighted in accordance with a frequency. A traveling time for which the vehicle is traveling is detected. Motion sickness of the occupant (more strictly, an accumulation factor of motion sickness) is integrally calculated using Expression (1) above described in ISO 2631.

It should be noted that the head motion of the occupant can also be obtained by either one of the methods (1) and (2) below other than such simulation calculation.

(1) An actual head motion is detected using an occupant's video obtained by imaging the occupant.
(2) A head motion is estimated from a traveling route of the vehicle and an actually detected motion in a camera video.

Moreover, recovery of the motion sickness of the occupant also occurs over time while the vehicle is traveling. The recovery occurrence of the motion sickness is approximated as a linear function on the basis of experimental results. In view of this, in the present disclosure, the motion sickness is predicted in consideration of both the accumulation factor and the recovery factor of the motion sickness on the basis of experimental results.

The MSDV described in ISO is a motion sickness index calculated by time integration of the head motion and can be said to be the accumulation factor of the motion sickness. On the other hand, a simulator sickness questionnaire (SSQ) is known as a subjective evaluation value of proneness to motion sickness. The SSQ is widely used as a questionnaire to determine a motion sickness level. Specifically, four items, nausea, oculomotor, disorientation, and total score, are calculated and evaluated by weighting scores obtained from answering 16 questions on a 4-point scale from 0 to 3. ISO 2631 has clearly described that there is a constant correlation between the MSDV and the proneness to motion sickness, SSQ, obtained by subjective evaluation. That is, provided that the horizontal axis is the MSDV and the vertical axis is the SSQ, a constant relationship is established, where the higher the MSDV is, the higher the SSQ is, and people are more prone to motion sickness.

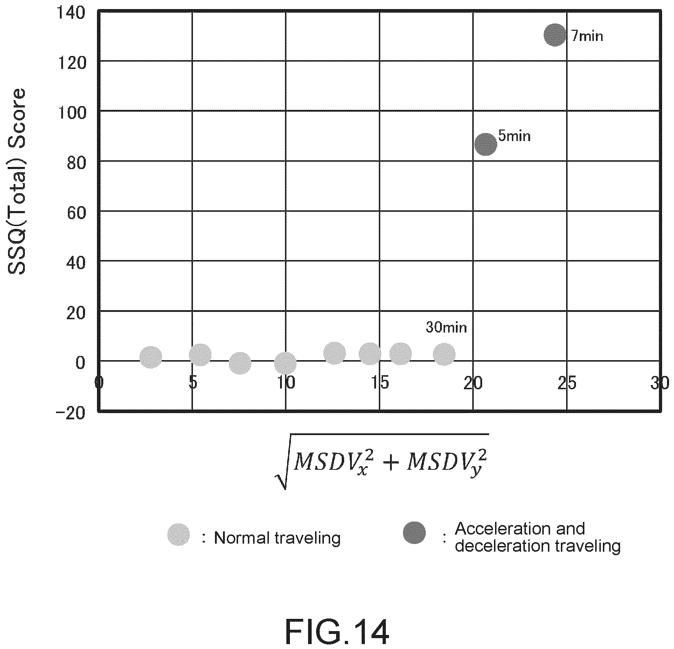

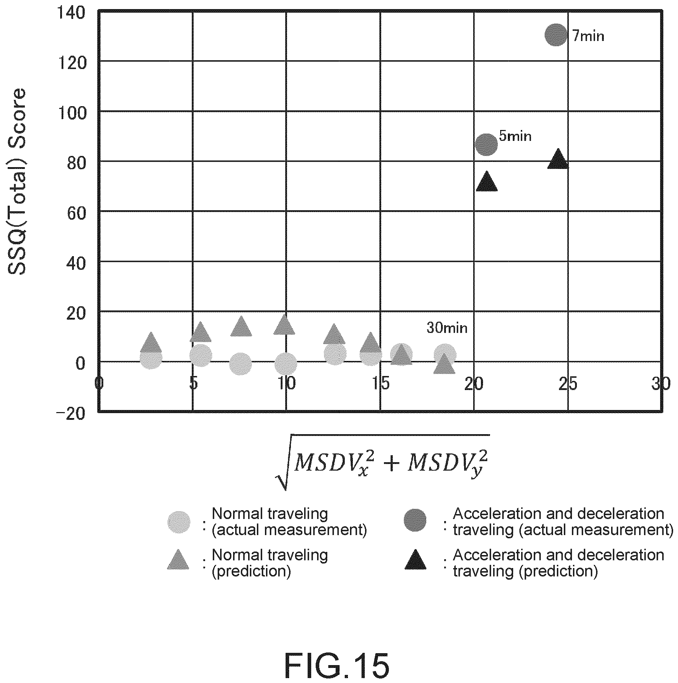

Fig. 14 shows experimental results of the MSDV and the SSQ in a case where the vehicle is normally traveling (smooth driving) and in a case where the vehicle is traveling to be accelerated and decelerated (hard driving), i.e., in cases where how the vehicle is traveling is different. Even in a case where the vehicle is normally traveling (smooth driving), the MSDV increases over time because it is obtained by time integration of the head motion. In contrast, the SSQ does not increase because the recovery of motion sickness occurs in accordance with elapse of time during the normal driving. Fig. 14 shows a state in which only the MSDV increases and the SSQ does not significantly change in a case where the normal driving has been continued for 30 minutes. On the other hand, in a case where the vehicle is traveling to be accelerated and decelerated (hard driving), the MSDV obtained by time integration of the head motion increases and the SSQ also increases over time because no recovery of the motion sickness occurs. Fig. 14 shows a state in which during the acceleration and deceleration driving, the MSDV and the SSQ both increase after 5 minutes and the MSDV and the SSQ also further increase after 7 minutes. For reference, Fig. 15 shows predicted MSDV and SSQ according to how the vehicle is traveling in an overlap manner. Also in the prediction results, as in the actual measurement, the MSDV obtained by time integration increases while the SSQ does not increase due to the recovery of the motion sickness during the normal driving. On the other hand, the MSDV and the SSQ both increase over time during the acceleration and deceleration driving.

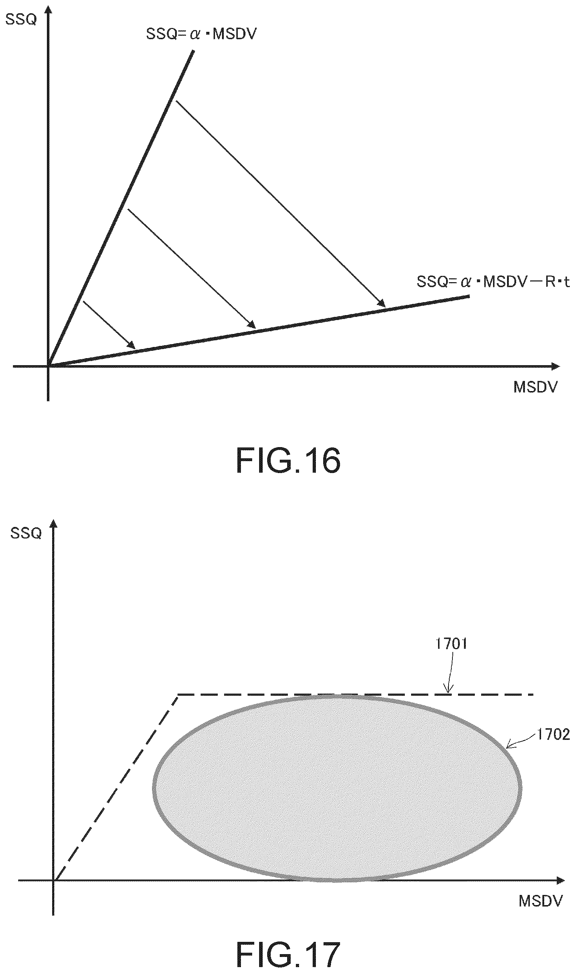

Fig. 16 schematically shows a relationship between the MSDV and the SSQ in each case, during the acceleration and deceleration driving or during the normal driving, which is derived from Figs. 14 and 15. During the acceleration and deceleration driving (hard driving), the recovery of the motion sickness does not occur (or is not significant), and therefore the relationship, SSQ = $\alpha$*MSDV, is established (It should be noted that $\alpha$ denotes a correlation coefficient of the MSDV and the SSQ). Moreover, during the normal driving (smooth driving), the recovery of the motion sickness (R*t) occurs over the time t, and therefore the relationship, SSQ = $\alpha$*MSDV - R*t, is established. Then, in accordance with how the vehicle is traveling, the relationship between the MSDV and the SSQ is established between SSQ = $\alpha$*MSDV and SSQ = $\alpha$*MSDV - R*t.

As shown in Fig. 17, it is possible to cope with prediction of the motion sickness, considering that the motion sickness does not occur unless the SSQ is larger than a certain threshold (limit) 1701. While driving to avoid the motion sickness is performed, the MSDV and the SSQ are substantially distributed in a range shown as the reference numeral 1702. The MSDV can be calculated on the basis of driving of the vehicle. Therefore, the motion sickness can be predicted on the basis of whether or not the SSQ exceeds the threshold 1701 from the MSDV calculated on the basis of data measured by a vehicle-mounted acceleration sensor.

Moreover, when how the relationship between the motion sickness susceptibility questionnaire (MSSQ) and the SSQ that a person has changes for each time was checked, a correlation between the susceptibility and the SSQ was confirmed for 10 minutes or more and no correlation was confirmed for less than 10 minutes. Therefore, it can be said that measurement for 10 minutes or more is necessary or strongly desirable in order to improve the accuracy of motion sickness prediction.

Then, when the motion sickness of the occupant is predicted while considering the recovery factor through the simulation calculation of the head motion of the occupant and the calculation of the MSDV, it can contributes to response processing such as control of the vehicle motion (e.g., driving instruction) and notification to the occupant or driver.

A method of instructing the vehicle on the basis of the predicted motion sickness can include the following three examples.

(1) Case of Manual Driving

When the SSQ approaches the threshold, the driver is notified of the fact that the motion sickness of the occupant is more likely to occur. For example, the guidance may be voice guidance such as "reduce speed change" or "reduce sudden steering" to reduce the SSQ, or the guidance may be notified by lighting up a display screen on an instrument panel. In addition, voice guidance or route change linked to route information of a car navigation system may be performed. Moreover, the notification to the driver may be performed on an area easy to view via a display UI such as a head-up display.

(2) Case of Automatic Driving

When the SSQ approaches the threshold, automated driving is linked to the prediction of the motion sickness by limiting the traveling speed or limiting the acceleration, such that the SSQ can be reduced. Depending on a case, the route change (change to a flat route with less curves so that the SSQ can be reduced) is performed. The motion sickness is further predicted on the basis of a congestion status, and it is also fed back to the current driving.

(3) Field-of-View Control of Occupant

If the occupant is enabled to easily view the outside through a car window or the like, the occupant can easily recognize car behaviors. In view of this, when the SSQ approaches the threshold, field-of-view control of the occupant may be started. For example, a display system that displays a content video on a screen with adjustable transmittance in a cabin is mounted, and in a case where the SSQ approaches the threshold while the occupant is viewing the video, the transmittance of the screen is increased, or the display of the content video is reduced in size or moved to a periphery of the screen, or the transparency is increased by decreasing the contrast. Such field-of-view control lowers the visibility of the content, but the occupant can easily look around the outside of the vehicle and can easily recognize car behaviors, such that the SSQ can be reduced. It should be noted that the display system with the screen with adjustable transmittance may be, for example, a combination of a projector and a transparent screen or is constituted by, for example, a display device with a transparent flat panel display (FPD) such as a transparent organic light-emitting diode (OLED) or a transparent liquid crystal display (LCD). The screen of the display system may be arranged on back, front, left, and right car windows and a wall surface of the cabin or may be a projection screen or a display monitor flipped down from the cabin ceiling.

C. Recovery factor of Motion Sickness

As described above, in the present disclosure, the motion sickness is predicted in consideration of both the accumulation factor and the recovery factor of motion sickness. In this Item C, a use case and a modified example in which the accumulation factor of motion sickness can be considered to be important will be described.

C-1. Case of Shifting from City Driving to Highway Driving

In city driving where stop-and-go at traffic lights and the like and right and left turns at intersections are often performed, back-and-forth acceleration and deceleration and left and right movement are often performed. Then, in highway driving, it switches to driving at a substantially constant speed where steering is not frequently performed. In the city driving, frequent head motions are made, and therefore the motion sickness accumulates. On the other hand, in the highway driving, head motions are less due to less changes in speed or route, and therefore the motion sickness of the occupant decreases. Otherwise, on a highway, it switches to automated driving and the occupant can be relaxed, and therefore the motion sickness is reduced.

In the conventional method of calculating the motion sickness index by time integration of the head motion of the occupant, only the accumulation factor of the motion sickness is considered, and therefore even when the occupant is relaxed on the highway, it is predicted that the occupant suffers from motion sickness. As a result, the speed, acceleration, steering, and the like of the vehicle may be unnecessarily limited and an unnecessary route change may be performed. In this regard, in the present disclosure, the motion sickness is predicted in consideration of both the accumulation factor and the recovery factor of motion sickness, and therefore the motion sickness can be more correctly predicted in consideration of the possibility in that the occupant is relaxed on the highway.

For example, by analyzing a GPS signal received from a GPS satellite, measuring the current location of the vehicle, and further detecting map information of a driving planed route of the vehicle, whether the vehicle is traveling in a city or on a highway can be determined. Moreover, whether the vehicle is traveling in a city or on a highway can also be determined by performing image recognition or image analysis from a captured image of a vehicle-mounted camera.

C-2. Case Where Vehicle is Automatically Driven at Very Low Speed

For example, in a case where the vehicle is automatically driven at a very low speed like a compact vehicle for one person, it is basically smoothly driven, and therefore the motion sickness hardly occurs. However, if the motion sickness index is calculated by time integration of the head motion as in the conventional method, the motion sickness is always predicted after a certain time. In this regard, in the present disclosure, the motion sickness is predicted also considering the recovery factor, and therefore the motion sickness can be more correctly predicted in consideration of the possibility in that the occupant is relaxed due to the smooth driving.

C-3. Changes in Motion Sickness Recovery Degree due to Orientations of Front Passenger Sheet and Second-Row and Third-Row Sheets

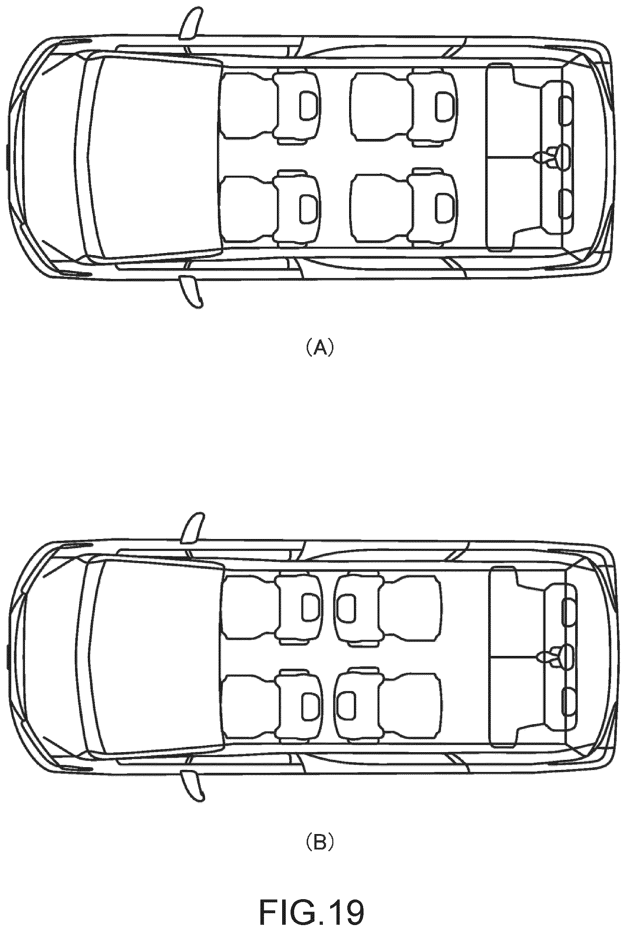

Fig. 19 illustrates an overhead view of a cabin of a 3-row seat car. It should be noted that in Fig. 19 (A), the seats of the three rows all face forwards. Meanwhile, in Fig. 19 (B), the seats of the second row have been turned, facing the seats of the third row. It can be seen also from Fig. 19 that a vehicle front field-of-view varies depending on which of the row of seats the occupant sits in, i.e., for each seat position. Moreover, up and down, left and right motions of the seat surface are not uniform during the driving, and the head motion varies depending on the occupant's sitting position. Therefore, it can be said that even in the interior of the same vehicle, a recovery degree of the motion sickness varies depending on the occupant's sitting position.

For example, on the front passenger seat, the front field-of-view is wide, and therefore car behaviors can be easily recognized. Thus, the recovery effect of the motion sickness is large. On the other hand, the field-of-view information decreases on the rear seat and the seat behind it, and therefore the car behaviors cannot be easily recognized. Thus, the recovery effect of the motion sickness is small correspondingly. Moreover, in a case where the seat has been turned facing backwards, the field-of-view information in front of the vehicle is lost, and it is thus difficult to recognize car behaviors. Thus, the recovery effect of the motion sickness decreases correspondingly.

In view of this, in the present disclosure, the position of the occupant is detected by the use of the in-vehicle camera, the body pressure sensor, and the like to grasp which of the row of seats the occupant sits in. Then, in consideration of the fact that the recovery effect of the motion sickness becomes smaller on the seat of the rear row such as the second or third row than on the first row, weighting calculation is performed on the recovery factor in accordance with a boarding position, and predicted motion sickness at the boarding position is calculated. Moreover, as shown in Fig. 19 (B), when the occupant sits facing backwards in an opposing seats-type or the like, it is difficult to recognize car behaviors, losing the field-of-view information in front of the vehicle, and it is thus difficult to recover from the motion sickness. In view of this, weighting calculation is performed on the recovery factor also in consideration of the posture of the occupant such as seat orientation in addition to the boarding position, and the predicted motion sickness is more correctly calculated.

C-4. Changes in Motion Sickness Recovery Degree due to Content Display Control

In a case where a display system that displays a content video on a screen with adjustable transmittance is mounted on the cabin and the occupant views the video, field-of-view information that can be acquired through the screen changes depending on the screen transmittance and the display size and the display position of the content video, and therefore the recovery degree of the motion sickness changes. For example, in a case where the transmittance of the screen is high, the size of the content video is small, or the content video is displayed at a position other than the center of the screen, the field-of-view of the occupant is wide and car behaviors can be easily recognized, and therefore the recovery effect of the motion sickness is large.

In view of this, in the present disclosure, when the screen transmittance and the display size and the display position of the content video are acquired by inquiring the display system, the field-of-view of the occupant information is grasped and weighting calculation is performed on the recovery factor on the basis of the easiness to recognize car behaviors and predicted motion sickness according to a display status of the content video is calculated.

D. System Configuration and Operation

In this Item D, the system configuration and operation in a case where the present disclosure is applied to the vehicle will be described.

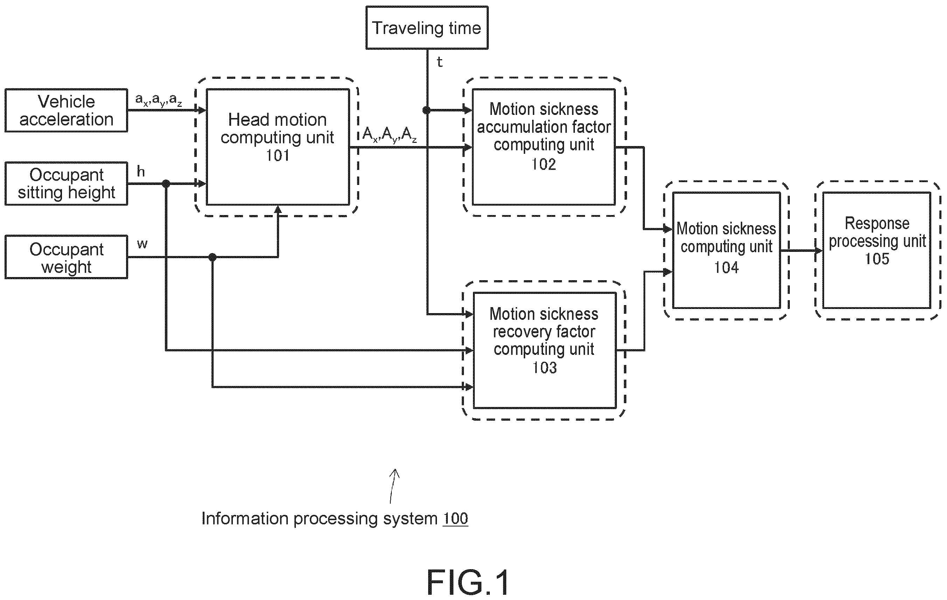

Fig. 1 schematically shows a functional configuration of an information processing system 100 that estimates the motion sickness of the occupant in the vehicle. The information processing system 100 shown in the figure includes a head motion computing unit 101, a motion sickness accumulation factor computing unit 102, a motion sickness recovery factor computing unit 103, a motion sickness computing unit 104, and a response processing unit 105. The information processing system 100 may be configured as a single apparatus with a physically single casing in which those functional blocks 101 to 105 are all stored. However, the head motion computing unit 101, the motion sickness accumulation factor computing unit 102, the motion sickness recovery factor computing unit 103, the motion sickness computing unit 104, the response processing unit 105 may be configured as respective individual apparatuses (in Fig. 1, the functional blocks that can be configured as the respective individual apparatuses are surrounded by the dotted line). Moreover, the functional blocks 101 to 105 may be classified into two or more groups and each group may be configured as an apparatus. Specifically, they may be configured as a physically single apparatus storing the head motion computing unit 101, the motion sickness accumulation factor computing unit 102, the motion sickness recovery factor computing unit 103, and the motion sickness computing unit 104 in a single casing. Alternatively, while the head motion computing unit 101 and the response processing unit 105 are mounted on the vehicle, the motion sickness accumulation factor computing unit 102, the motion sickness recovery factor computing unit 103, and the motion sickness computing unit 104 may be configured as an external apparatus of the vehicle.

Such an information processing system 100 can be constituted by, for example, a personal computer (PC). Moreover, the respective components 101 to 104 of the information processing system 100 may be dedicated hardware circuits or may be realized in the form of executing predetermined software programs in the PC. Moreover, the respective components 101 to 104 may be all implemented in a single PC or may be arranged, distributed to two or more PCs.

The head motion computing unit 101 computes the head motion of the occupant on the basis of respective data detected by the in-vehicle sensors. Basically, the head motion computing unit 101 calculates a head motion of the occupant by simulation calculation on the basis of the motion of the vehicle. Specifically, the head motion computing unit 101 calculates head acceleration ($A_x$, $A_y$, $A_z$) of the occupant by simulation calculation on the basis of vehicle acceleration ($a_x$, $a_y$, $a_z$) detected by the acceleration sensor mounted on the rigid part of the vehicle and the attribute data of the occupant (body information such as a sitting height (h) and a weight (w) of an upper half of the body) detected from the in-vehicle monitoring camera and the body pressure sensor mounted on the seat surface. It should be noted that the head motion computing unit 101 may be adapted to detect an actual head motion from a captured image of the occupant and estimate a head motion from a traveling route of the vehicle and a motion in a camera video other than the above-mentioned simulation calculation.

The motion sickness accumulation factor computing unit 102 performs weighting on the head acceleration ($A_x$, $A_y$, $A_z$) calculated by the head motion computing unit 101 in accordance with a frequency, detects a traveling time for which the vehicle is traveling, and integrally calculates an accumulation factor of motion sickness of the occupant by time integration using Expression (1) above, which has been described in ISO 2631.

The motion sickness recovery factor computing unit 103 calculates a recovery factor of the motion sickness of the occupant on the basis of the detected attribute data (ditto) of the occupant and the detected traveling time of the vehicle.

The motion sickness computing unit 104 combines the accumulation factor of the motion sickness calculated by the motion sickness accumulation factor computing unit 102 and the accumulation factor of motion sickness calculated by the motion sickness recovery factor computing unit 103 to calculate motion sickness of the occupant.

The response processing unit 105 performs processing to recover from the motion sickness of the occupant in accordance with a result of the motion sickness of the occupant which is calculated by the motion sickness computing unit 104. Specifically, the response processing unit 105 performs response processing such as notification to the occupant or driver in manual driving (e.g., instruction of driving operation or route change for reducing the SSQ of the occupant), driving control in automated driving (e.g., driving control or route change for limiting acceleration and deceleration or route change for reducing the SSQ of the occupant), and field-of-view control of the occupant (display control of the content video in the display system mounted on the interior of the vehicle).

It should be noted that details of the respective components 101 to 104 will be described later.

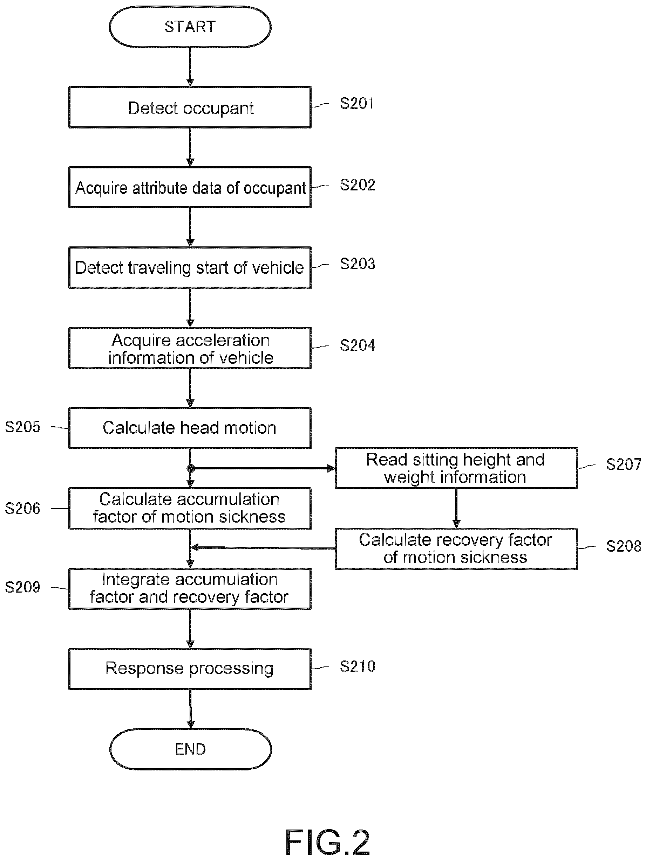

Fig. 2 shows a general operation procedure performed on the information processing system 100 as a flowchart.

When a person appears in the vehicle, i.e., an occupant has been detected (Step S201), first of all, attribute data of the occupant is acquired (Step S202). Specifically, body information such as a sitting height (h) and a weight (w) of the upper half of the body of the occupant is acquired by the use of the in-vehicle sensors such as the in-vehicle monitoring camera and the body pressure sensor mounted on the seat surface.

Then, when it has been detected that the vehicle has started driving (Step S203), vehicle acceleration ($a_x$, $a_y$, $a_z$) is detected by the use of the acceleration sensor mounted on the rigid part of the vehicle (Step S204).

Subsequently, the head motion computing unit 101 calculates head acceleration ($A_x$, $A_y$, $A_z$) of the occupant by simulation calculation on the basis of the vehicle acceleration information acquired in Step S203 and the body information such as the sitting height (h) and the weight (w) of the upper half of the body of the occupant which are acquired in Step S202 (Step S205).

Subsequently, the motion sickness accumulation factor computing unit 102 performs weighting on the head acceleration ($A_x$, $A_y$, $A_z$) calculated by the head motion computing unit 101 in accordance with a frequency, detects a traveling time for which the vehicle is traveling, and integrally calculates an accumulation factor M of the motion sickness of the occupant by time integration using Expression (1) above, which has been described in ISO 2631 (Step S206).

Moreover, the motion sickness recovery factor computing unit 103 reads the attribute data of the occupant including the body information such as the sitting height (h) and the weight (w) of the upper half of the body of the occupant which are acquired in Step S202 (Step S207) and performs computing processing of a recovery factor R of the motion sickness of the occupant on the basis of an elapsed time (Step S208).

Then, the motion sickness computing unit 104 combines an accumulation factor $\alpha M$ of the motion sickness which is calculated by the motion sickness accumulation factor computing unit 102 and the recovery factor R of the motion sickness calculated by the motion sickness recovery factor computing unit 103 to calculate motion sickness S ($= \alpha M - R$) of the occupant (Step S209).

The response processing unit 105 performs processing to recover from the motion sickness of the occupant in accordance with a result of the motion sickness of the occupant which is calculated by the motion sickness computing unit 104 (Step S210). For example, the response processing unit 105 performs response processing such as notification to the occupant or driver in manual driving, driving control in the automated driving, and field-of-view control of the occupant.

D-1. Simulation Calculation of Head Motion of Occupant

In this Item D-1, details of simulation calculation of the head motion performed by the head motion computing unit 101 will be described. The head motion computing unit 101 is a functional block that predicts the head motion of the occupant by simulation calculation on the basis of detected data.

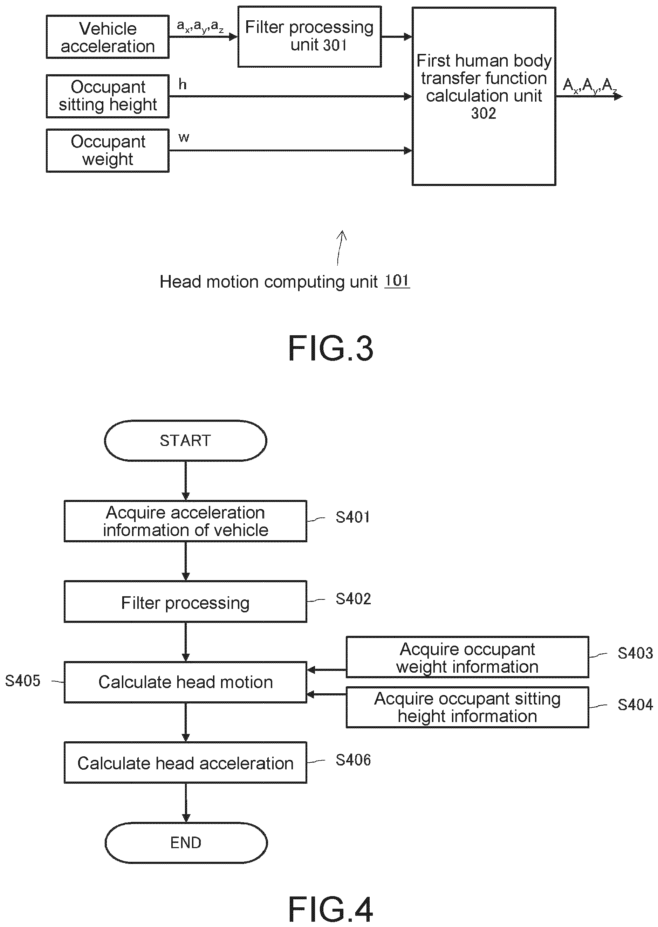

Fig. 3 shows an internal configuration example of the head motion computing unit 101. In the example shown in the figure, the head motion computing unit 101 includes a filter processing unit 301 and a first human body transfer function calculation unit 302.

The filter processing unit 301 performs canceling of fine noise included in the vehicle acceleration ($a_x$, $a_y$, $a_z$) directly output from the acceleration sensor mounted on the rigid part of the vehicle and filter processing for calculation processing load reduction.

The first human body transfer function calculation unit 302 inputs the filter-processed vehicle acceleration ($a_x$, $a_y$, $a_z$) and acquires, from the in-vehicle monitoring camera and the body pressure sensor mounted on the seat surface, body information such as a sitting height (h) and a weight (w) of the upper half of the body which are the attribute data of the occupant. Then, the first human body transfer function calculation unit 302 uses a simulation model of the human body (upper half of the body) to calculate head motion of the occupant (head acceleration ($A_x$, $A_y$, $A_z$)) by simulation on the basis of the motion of the vehicle (filter-processed vehicle acceleration ($a_x$, $a_y$, $a_z$)).

The simulation performed by the first human body transfer function calculation unit 302 is dynamics calculation in which the vehicle and the occupant are approximated as mechanical elements such as spring, mass, and damper and the above-mentioned three input parameters are used. It should be noted that the simulation model of the human body to be used is not particularly limited.

It should be noted that a method of calculating the head motion of the occupant other than the simulation can also include a method of using data based on previous experimental results and using a machine learning model that has learned detection results in advance for actual vehicle acceleration data and head motion according to the camera.

Fig. 4 shows a processing procedure of calculating the head motion of the occupant in the head motion computing unit 101 shown in Fig. 3 as a flowchart.

First of all, when vehicle acceleration ($a_x$, $a_y$, $a_z$) directly output from the acceleration sensor mounted on the rigid part of the vehicle is acquired (Step S401), the filter processing unit 301 performs canceling of fine noise included in the vehicle acceleration ($a_x$, $a_y$, $a_z$) and filter processing for calculation processing load reduction (Step S402).

Subsequently, the first human body transfer function calculation unit 302 acquires, from the in-vehicle monitoring camera and the body pressure sensor mounted on the seat surface, body information such as a sitting height (h) and a weight (w) of the upper half of the body which are the attribute data of the occupant (Step S403 and S404), calculates a head motion of the occupant by simulation using the simulation model of the human body (upper half of the body) on the basis of the motion of the vehicle (filter-processed vehicle acceleration ($a_x$, $a_y$, $a_z$)) (Step S405), and calculates head acceleration ($A_x$, $A_y$, $A_z$) of the occupant (Step S406).

D-2. Calculation for Accumulation factor of Motion Sickness

In this D-2 Item, details of calculation for the accumulation factor of the motion sickness which is executed by the motion sickness accumulation factor computing unit 102 will be described. The motion sickness accumulation factor computing unit 102 is a functional block that calculates the determination index MSDV of the motion sickness by time integration defined in ISO 2631.

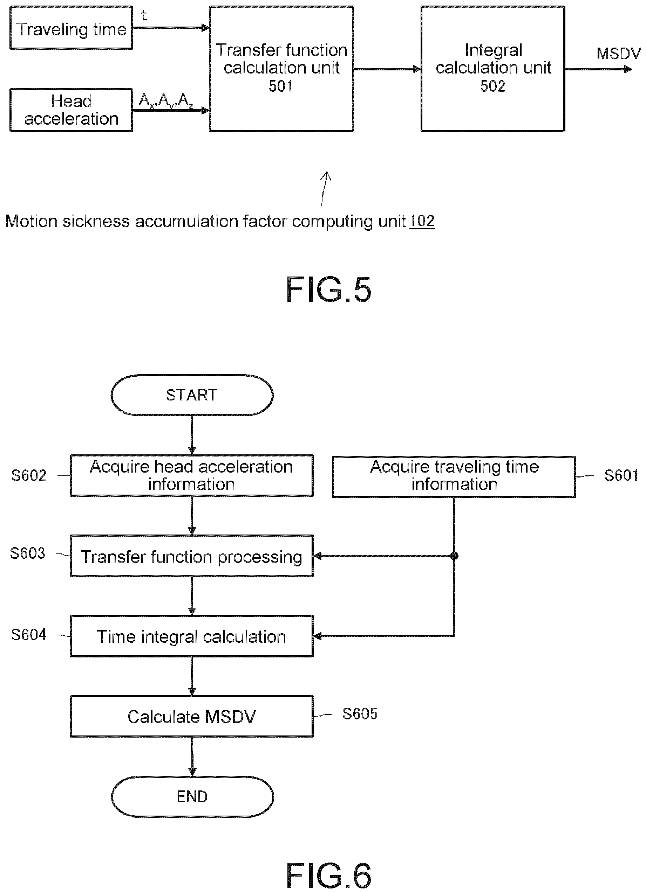

Fig. 5 shows an internal configuration example of the motion sickness accumulation factor computing unit 102. In the example shown in the figure, the motion sickness accumulation factor computing unit 102 includes a transfer function calculation unit 501 and an integral calculation unit 502.

The transfer function calculation unit 501 inputs traveling time information t and head acceleration ($A_x$, $A_y$, $A_z$) of the occupant calculated by the head motion computing unit 101, converts this head acceleration ($A_x$, $A_y$, $A_z$) from the time domain to the frequency domain temporarily, multiplies it by the transfer function H(s) of the filter, and then inversely converts it to the time domain again to calculate weighted effective acceleration (see Fig. 13). As described above in Item B, the filter characteristics obtained by changing the coefficients $f_i$ (= $\omega_i$) and $Q_i$ in accordance with the motion (e.g., vehicle type) are used as the transfer function H(s).

Using Expression (1) above, the integral calculation unit 502 squares the weighted effective acceleration calculated by the transfer function calculation unit 501, integrates it over time, and performs route calculation, thereby calculating one determination index MSDV of the motion sickness defined in ISO 2631 as the accumulation factor of the motion sickness.

Fig. 6 shows a processing procedure for calculating the accumulation factor of the motion sickness of the occupant in the motion sickness accumulation factor computing unit 102 shown in Fig. 5 as a flowchart.

First of all, traveling time information t is acquired (Step S601) and head acceleration ($A_x$, $A_y$, $A_z$) of the occupant calculated by the head motion computing unit 101 is acquired (Step S602).

The transfer function calculation unit 501 converts the head acceleration ($A_x$, $A_y$, $A_z$) from the time domain to the frequency domain temporarily, multiplies it by the transfer function H(s) of the filter, and then inversely converts it to the time domain again to calculate weighted effective acceleration (Step S603).

Subsequently, using Expression (1) above, the integral calculation unit 502 squares the weighted effective acceleration calculated by the transfer function calculation unit 501, integrates it over time, and performs route calculation (Step S604), thereby calculating one determination index MSDV of the motion sickness defined in ISO 2631 as the accumulation factor of the motion sickness (Step S605).

D-3. Calculation for Recovery factor of Motion Sickness

In this Item D-3, details of calculation for the accumulation factor of motion sickness which is executed by the motion sickness recovery factor computing unit 103 will be described. The motion sickness recovery factor computing unit 103 is a functional block that calculates the recovery effect of the motion sickness of the occupant on the basis of the occupant attribute data and the position and posture information of the occupant in the vehicle which are detected by the in-vehicle sensors and the like and the elapsed time. On the basis of the attribute data of the occupant that affects the recovery (e.g., the sitting height, weight of the occupant, the position and posture of the occupant in the vehicle), the motion sickness recovery factor computing unit 103 calculates an occupant attribute parameter value to be used for recovery calculation of the motion sickness calculates an effect R to recover from the motion sickness based on the calculated occupant attribute parameter value and an elapsed time t. As to calculation of the parameter values, it is possible to improve the calculation accuracy by using a machine learning model that has learned the occupant's boarding history in advance.

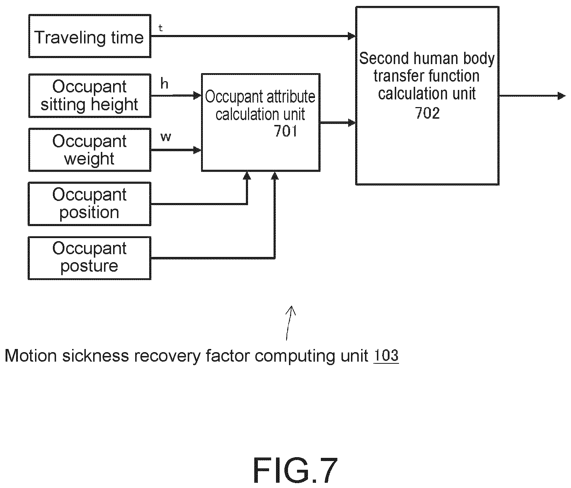

Fig. 7 shows an internal configuration example of the motion sickness recovery factor computing unit 103. In the example shown in the figure, the motion sickness recovery factor computing unit 103 includes an occupant attribute calculation unit 701 and a second human body transfer function calculation unit 702.

The occupant attribute calculation unit 701 inputs body information of the sitting height, the weight of the upper half of the body, and the like of the occupant and a state of the position and posture of the occupant in the vehicle, which are detected by the in-vehicle sensors such as the in-vehicle monitoring camera and the body pressure sensor mounted on the seat surface, and calculates an occupant attribute parameter value to be used for recovery calculation of the motion sickness. Specifically, a rate of change (gradient) of the amount of change over time at which a SSQ total score recovers (or lowers) when the input parameters are changed is determined by experiments in advance. Then, on the basis of the experimental results, the occupant attribute calculation unit 701 calculates the amount of change over time of the recovery of the motion sickness corresponding to the input attributes of the occupant as the occupant attribute parameter value. As to calculation of the parameter values, it is possible to improve the calculation accuracy by using a machine learning model that has learned the occupant's boarding history in advance.

The second human body transfer function calculation unit 702 multiplies the amount of change over time of the recovery of the SSQ total score determined by the occupant attribute calculation unit 701 by the actual traveling time, thereby calculating a recovery amount R of the motion sickness from the driving start.

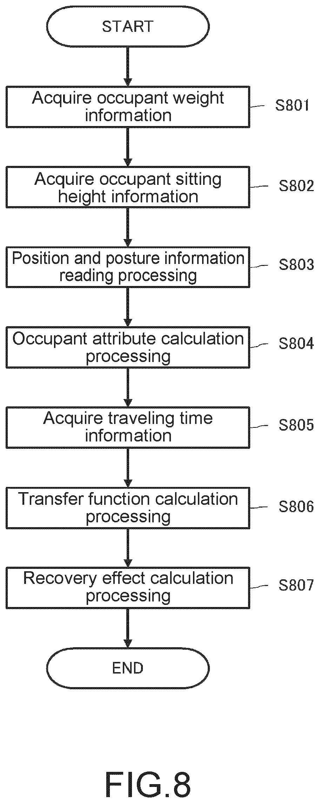

Fig. 8 shows a processing procedure for calculating the accumulation factor of motion sickness of the occupant in the motion sickness recovery factor computing unit 103 shown in Fig. 7 as a flowchart.

First of all, the occupant attribute calculation unit 701 acquires weight information w of the occupant from the body pressure sensor mounted on the seat surface of the seat in which the occupant sits (Step S801) and acquires sitting height information h of the occupant from an image captured by the in-vehicle monitoring camera or the like (Step S802). In addition, the occupant attribute calculation unit 701 reads, from the captured image or the like of the in-vehicle monitoring camera, information of the occupant's sitting position (which of the row of seats the occupant sits in) in the vehicle and a posture (Step S803).

Then, the occupant attribute calculation unit 701 calculates an occupant attribute parameter value to be used for recovery calculation of the motion sickness (Step S804). Specifically, a rate of change (gradient) of the amount of change over time at which the SSQ total score recovers (or lowers) by experiments are determined in advance by experiments. On the basis of the experimental results, the occupant attribute calculation unit 701 calculates the amount of change over time of the recovery of the motion sickness corresponding to the input parameters acquired in previous Step S801 to S803 as the occupant attribute parameter value.

Subsequently, information of the traveling time of the vehicle is acquired (Step S805). Then, the second human body transfer function calculation unit 702 multiplies the amount of change over time of the recovery of the SSQ total score which is determined by the occupant attribute calculation unit 701 by the actual traveling time (Step S806), thereby calculating a recovery amount R of the motion sickness from the driving start (Step S807).

D-4. Computing Operation for Motion Sickness

In this Item D-4, details of the motion sickness calculation executed by the motion sickness computing unit 104 will be described.

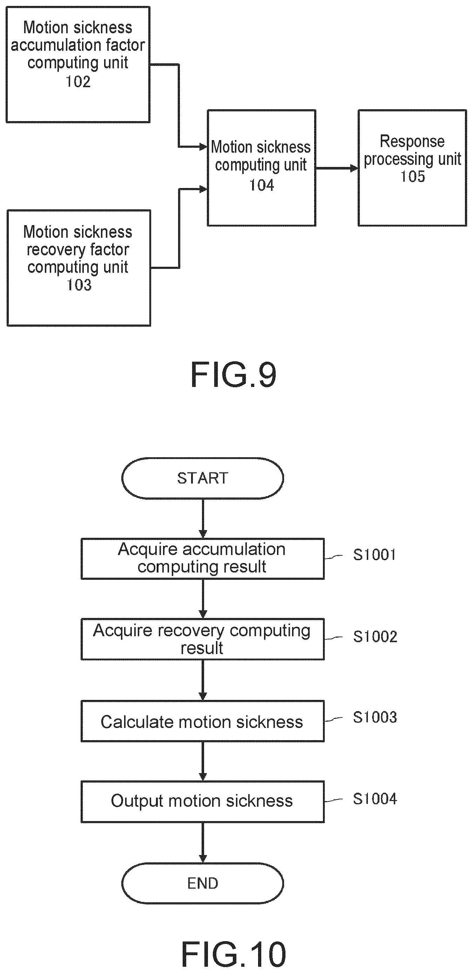

Fig. 9 shows the motion sickness computing unit 104, and an input/output part of the motion sickness computing unit 104 which are extracted from the overall configuration of the information processing system 100 shown in Fig. 1. As described above in Item D-2, the motion sickness accumulation factor computing unit 102 calculates a determination index MSDV based on time integration of the head motion, which is defined in ISO 2631, as the accumulation factor of the motion sickness. Moreover, as described above in Item D-3, the motion sickness recovery factor computing unit 103 calculates a recovery effect of the motion sickness of the occupant on the basis of body information such as the sitting height h, the weight w of the upper half of the body, and the like of the occupant, the position and posture information of the occupant in the vehicle, and the elapsed time t. Then, the motion sickness computing unit 104 combines the MSDV calculated by the motion sickness accumulation factor computing unit 102 and the recovery degree of the motion sickness calculated by the motion sickness recovery factor computing unit 103 to calculate motion sickness of the occupant.

Here, provided that the MSDV calculated by the motion sickness accumulation factor computing unit 102 is the accumulation factor M of the motion sickness and R denotes the recovery degree of the motion sickness calculated by the motion sickness recovery factor computing unit 103, the motion sickness computing unit 104 subtracts the recovery degree R of the motion sickness from the accumulation factor M of the motion sickness as shown in Expression (2) below to calculate motion sickness S of the occupant. It should be noted that in this Expression (2), the coefficient $\alpha$ by which the accumulation factor M is multiplied is the correlation coefficient of the MSDV and the SSQ determined from experiments.
[Formula 2]

$$S = \alpha M - R \qquad \cdots (2)$$

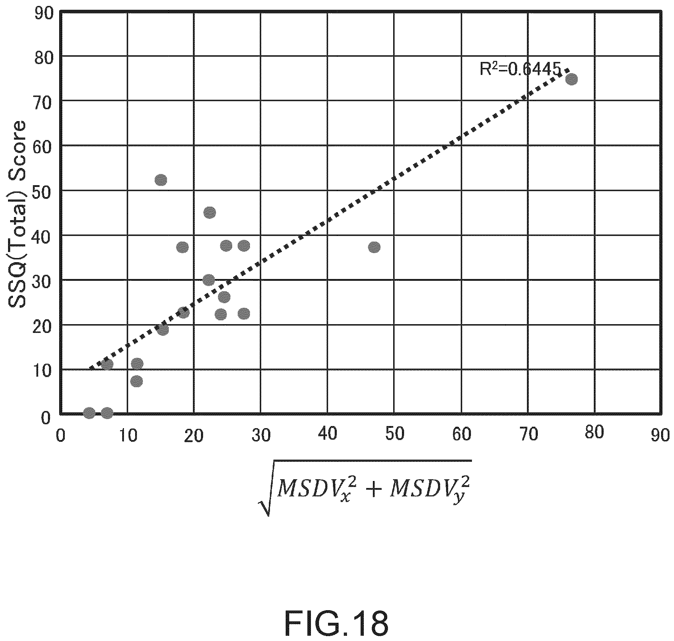

It should be noted that the correlation coefficient $\alpha$ of the MSDV and the SSQ varies depending on how the vehicle is traveling. For example, the correlation coefficient $\alpha$ differs between the acceleration and deceleration driving (hard driving) and the normal driving (smooth driving) and the correlation coefficient differs also in the city driving and the highway driving. Fig. 18 shows an example of experimental results of the MSDV and the SSQ total score when the vehicle performs the same driving. In experimental results shown in the figure, the coefficient of determination (square of the correlation coefficient) $R^2$ is 0.6445.

Fig. 10 shows a processing procedure for calculating the motion sickness of the occupant in the motion sickness computing unit 104 shown in Fig. 9 as a flowchart.

First of all, the motion sickness computing unit 104 acquires the determination index MSDV based on time integration of the head motion, which is calculated by the motion sickness accumulation factor computing unit 102 (Step S1001) and acquires the recovery effect of the motion sickness of the occupant calculated by the motion sickness recovery factor computing unit 103 on the basis of the occupant attribute information (Step S1002). It should be noted that although in the flowchart shown in Fig. 10, Step S1001 of computing the accumulation factor of the motion sickness and Step S1002 of computing the accumulation factor of motion sickness are arranged in chronological order in the stated order, Step S1001 and Step S1002 may be executed in parallel or may be executed in the opposite order.

Subsequently, the motion sickness computing unit 104 combines the MSDV (M) calculated by the motion sickness accumulation factor computing unit 102 and the recovery degree (R) of the motion sickness calculated by the motion sickness recovery factor computing unit 103 in accordance with Expression (2) above to calculate motion sickness (S) of the occupant (Step S1003).

Then, the motion sickness computing unit 104 outputs the calculated motion sickness (S) of the occupant to the response processing unit 105 at the subsequent stage (Step S1004) and terminates this processing.

D.5. Changes in Motion Sickness Recovery Degree due to Orientations of Front Passenger seat and Second-Row and Third-Row Seats

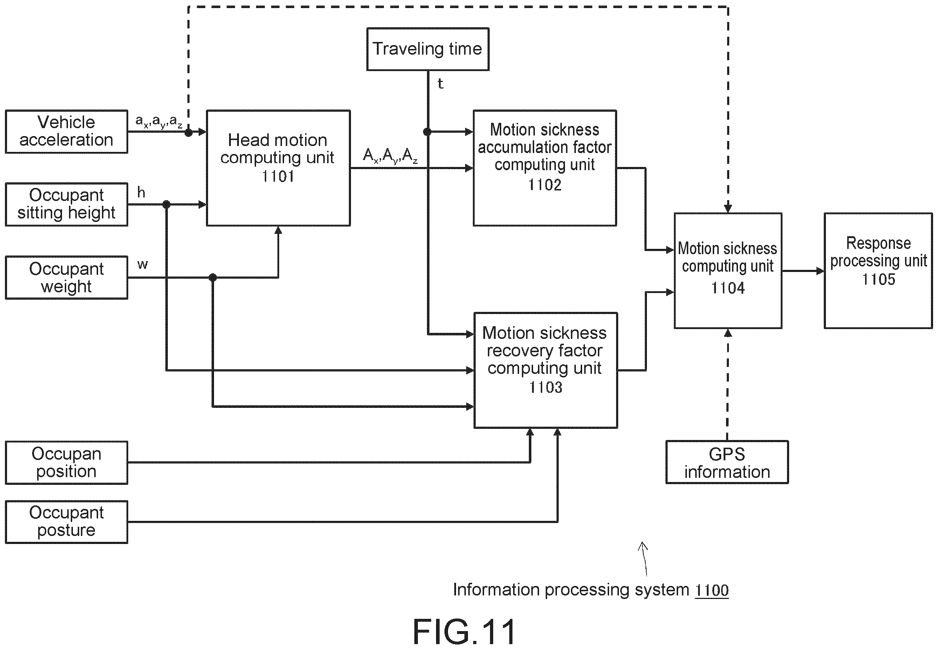

Fig. 11 schematically shows a functional configuration of an information processing system 1100 that estimates the motion sickness of the occupant. The information processing system 1100 shown in Fig. 11 is different from the information processing system 100 shown in Fig. 1 in that it estimates the motion sickness also in consideration of the position of the occupant in the vehicle and the posture of the occupant. The information processing system 1100 includes a head motion computing unit 1101, a motion sickness accumulation factor computing unit 1102, a motion sickness recovery factor computing unit 1103, a motion sickness computing unit 1104, and a response processing unit 1105.

On the basis of respective data detected by the in-vehicle sensors, the head motion computing unit 1101 calculates a head motion of the occupant by simulation calculation on the basis of the motion of the vehicle.

The motion sickness accumulation factor computing unit 1102 performs weighting on the head acceleration ($A_x$, $A_y$, $A_z$) calculated by the head motion computing unit 1101 in accordance with a frequency, detects a traveling time for which the vehicle is traveling, and integrally calculates an accumulation factor MSDV of motion sickness of the occupant by time integration using Expression (1) above, which has been described in ISO 2631.

The motion sickness recovery factor computing unit 1103 calculates a recovery factor of the motion sickness of the occupant also in consideration of the position of the occupant in the vehicle and the posture of the occupant in addition to the detected attribute data (ditto) of the occupant and the traveling time of the vehicle. The position of the occupant in the vehicle and the posture of the occupant are acquired for example on the basis of an image captured by the in-vehicle monitoring camera. Moreover, as described above in Item C-3, the recovery degree of the motion sickness changes depending on the occupant's sitting position even in the interior of the same vehicle. It is because for example in a case of a vehicle in which seats of three or more rows are installed, the front field-of-view and up and down, left and right motions are not uniform for each seat position, and the head motion varies depending on the occupant's sitting position. In consideration of the fact that the recovery effect of the motion sickness becomes smaller on the seat of the rear row such as the second or third row than on the first row and also in consideration of the recovery effect according to the posture (seat orientation) of the occupant, the motion sickness recovery factor computing unit 1103 performs weighting calculation on the recovery factor in accordance with the boarding position and the posture of the occupant to calculate predicted motion sickness at the boarding position.

Specifically, the SSQ total score when the position and the posture of the occupant are changed is determined by experiments in advance, and the score ratio under each condition is calculated using "the state in which the occupant in the second row is sitting facing forwards" as a reference position. Then, the motion sickness recovery factor computing unit 1103 multiplies the determined score ratio by the recovery calculation and reflects the effect according to the position and the posture of the occupant on a motion sickness recovery factor. Provided that $\beta$ denotes the SSQ total score ratio according to the position and the posture of the occupant, as shown in Expression (3) below, a value R' obtained by multiplying the recovery factor R of the motion sickness calculated on the basis of the body information such as the sitting height h and the weight w of the upper half of the body of the occupant and the elapsed time t by $\beta$ is the motion sickness recovery factor reflecting the effect according to the position and the posture of the occupant.

[Formula 3]

$$R' = R \times \beta \qquad \cdots (3)$$

The motion sickness computing unit 1104 combines the accumulation factor of the motion sickness calculated by the motion sickness accumulation factor computing unit 1102 and the accumulation factor of motion sickness calculated by the motion sickness recovery factor computing unit 103 to calculate motion sickness of the occupant. That is, as shown in Expression (4) below, the motion sickness computing unit 1104 subtracts the motion sickness recovery factor R' reflecting the effect according to the position and the posture of the occupant from a value $\alpha M$ obtained by multiplying the MSDV calculated by the motion sickness accumulation factor computing unit 102 by the correlation coefficient $\alpha$ of the MSDV and the SSQ to calculate motion sickness S of the occupant.

[Formula 4]

$$S = \alpha M - R' \qquad \cdots (4)$$

Moreover, as also described in Item D-4 above, the correlation coefficient $\alpha$ of the MSDV and the SSQ differs depending on how the vehicle is traveling. For example, the correlation coefficient $\alpha$ differs between the acceleration and deceleration driving (hard driving) and the normal driving (smooth driving) and the correlation coefficient also differs between the city driving and the highway driving. The motion sickness computing unit 1104 may be adapted to input the vehicle acceleration and determine how to travel (acceleration and deceleration driving (hard driving) or normal driving (smooth driving)) or input the GPS information and determine whether the vehicle is traveling in a city or on a highway and calculates motion sickness S of the occupant by using a suitable correlation coefficient $\alpha$ on the basis of a determination result.

The response processing unit 1105 performs processing according to a result of the motion sickness of the occupant which is calculated by the motion sickness computing unit 1104. Specifically, the response processing unit 1105 performs the response processing (ditto) of the notification to the occupant or driver in manual driving (e.g., instruction of driving operation or route change for reducing the SSQ of the occupant), driving control in automated driving (e.g., driving control or route change for limiting acceleration and deceleration or route change for reducing the SSQ of the occupant), and field-of-view control of the occupant (display control of the content video in the display system mounted on the interior of the vehicle).

Fig. 12 shows an operation procedure performed in the information processing system 1100 as a flowchart.

When a person appears in the vehicle, i.e., an occupant has been detected (Step S1201), first of all, attribute data of the occupant is acquired (Step S1202). Specifically, body information such as a sitting height (h) and a weight (w) of the upper half of the body of the occupant is acquired by the use of the in-vehicle sensors such as the in-vehicle monitoring camera and the body pressure sensor mounted on the seat surface.

Then, when it has been detected that the vehicle has started driving (Step S1203), vehicle acceleration $(a_x, a_y, a_z)$ is detected by the use of the acceleration sensor mounted on the rigid part of the vehicle (Step S1204).

Subsequently, the head motion computing unit 1101 calculates head acceleration $(A_x, A_y, A_z)$ of the occupant by simulation calculation on the basis of the vehicle acceleration information acquired in Step S1203 and the body information such as the sitting height (h) and the weight (w) of the upper half of the body of the occupant which are acquired in S1202 (Step S1205).

Subsequently, the motion sickness accumulation factor computing unit 1102 performs weighting on the head

acceleration ($A_x$, $A_y$, $A_z$) calculated by the head motion computing unit 1101 in accordance with a frequency, detects a traveling time for which the vehicle is traveling, and integrally calculates an accumulation factor M of the motion sickness of the occupant by time integration using Expression (1) above, which has been described in ISO 2631 (Step S1206).

Moreover, the motion sickness recovery factor computing unit 1103 reads the attribute data of the occupant including the body information such as the sitting height (h) and the weight (w) of the upper half of the body of the occupant which are acquired in Step S1202 (Step S1208) and performs computing processing of the accumulation factor R' of motion sickness reflecting the effect according to the position and the posture of the occupant (Step S1209).

Then, the motion sickness computing unit 1104 combines the accumulation factor $\alpha M$ of the motion sickness which is calculated by the motion sickness accumulation factor computing unit 1102 and the accumulation factor R' of motion sickness which is calculated by the motion sickness recovery factor computing unit 1103 to calculate motion sickness S of the occupant (= $\alpha M$ - R') (Step S1210).

The response processing unit 1105 performs response processing of notification to the occupant or driver, feedback to automated driving control, field-of-view control of the occupant (e.g., display control of content in an in-vehicle display system) in accordance with a result of the motion sickness of the occupant which is calculated by the motion sickness computing unit 1104 (Step S1211).

E. Display System and Control Thereof E-1. Configuration of Display System

There are needs to view a video on a large display in order to obtain a sense of immersion in the vehicle. For example, at Level 4 and Level 5 of automated driving, needs for the occupant to view a video increase. In view of this, in the present disclosure, it is envisaged that a display system that displays a content video in a transmittance variable region is mounted on the vehicle. The display system is also one of processing targets of the above-mentioned response processing unit 105 (or 1105).

Specifically, the display system is constituted by a combination of a transparent screen and a projector that projects a video on the transparent screen and displays a content video in a transmittance variable region. Here, the transparent screen is configured by, for example, laminating a film with electrically variable transmittance on a transparent film such as a resin. Moreover, the display system that displays the content video in the transmittance variable region may be configured with a display device or the like with a transparent FPD such as a transparent OLED or a transparent LCD. Alternatively, the display system may be of a video see-through type displays a landscape in the front of the vehicle (or the periphery of the vehicle), combined with a vehicle-mounted camera by using a display device of a non-see-through-type, not a see-through-type.

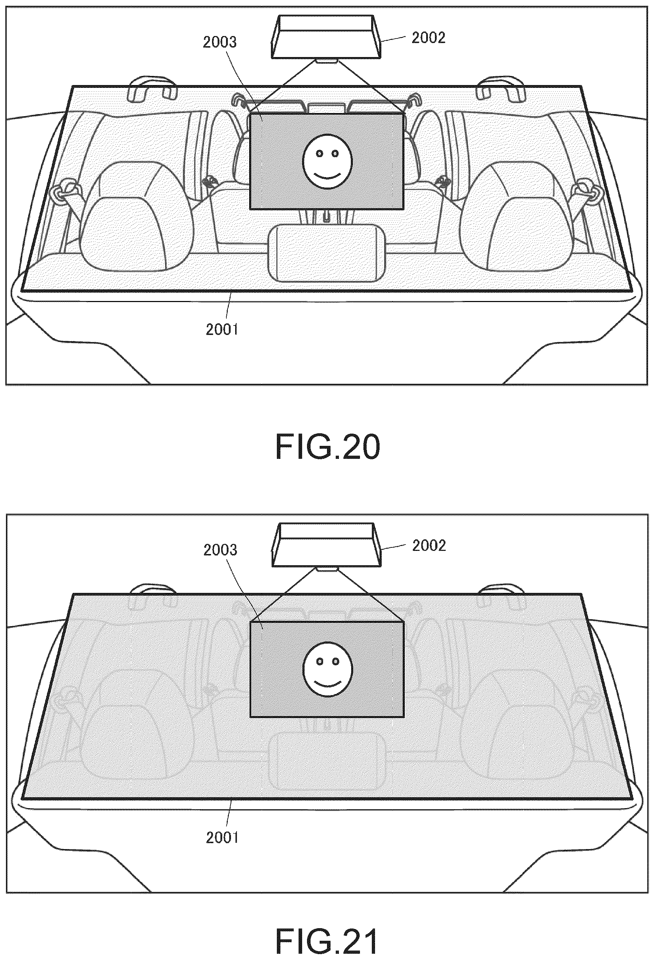

The transmittance variable region of the display system may be a transparent screen or a transparent FPD flipped down from the bottom surface of the cabin roof. Fig. 20 shows a state in which a transparent screen 2001 is flipped down from the bottom surface of the cabin roof and a content video 2003 is projected to substantially the center of the transparent screen 2001 from a projector 2002 mounted also on the bottom surface of the roof. The transparent screen 2001 of the flipped down type is mounted on the vicinity of the back of the seat of the first row, for example. Therefore, the occupant sitting in the rear seat can view the content video 2003 projected to the transparent screen 2001. In short, the display system shown in Fig. 20 can be said to be a rear-seats entertainment (RSE) system. It should be noted that it is assumed that the rear seat includes a seat of a second row of a 2-row seat car or a seat of second and third rows of a 3-row seat car.

A film with electrically variable transmittance is laminated on the transparent screen 2001, and by electrically controlling the transmittance of this film, it is possible to adjust the transmittance of the entire transparent screen 2001 or a part thereof (e.g., only a partial region in which the content video 2003 has been projected). Moreover, by adjusting the contrast of the content video 2003 projected by the projector 2002, it is possible to adjust the transmittance of the display region of the content video 2003 (increasing the irradiation intensity to increase the contrast decreases the transmittance and in contrast, decreasing the irradiation intensity to decrease the contrast increases the transmittance).

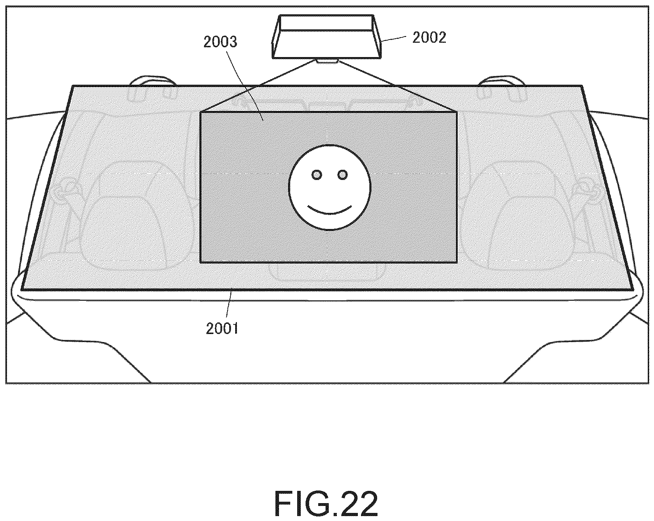

It can be seen that when the transmittance of the entire transparent screen 2001 or a part thereof is increased, the occupant in the rear seat can easily recognize the situation in the front of the vehicle and behaviors of the vehicle through its transparent region. On the other hand, as shown in Fig. 21, the sense of immersion of the content video 2003 increases when the transmittance of the entire transparent screen 2001 is decreased. As shown in Fig. 22, the sense of immersion further increases when the transmittance of the entire transparent screen 2001 is decreased and the display size of the content video 2003 is increased.

The same applies to a display system using a transparent FPD. By increasing the transmittance of the entire transparent FPD including the content video or increasing the transmittance of a region other than the display region of the content video, the occupant in the rear seat can easily recognize the situation in the front of the vehicle and behaviors of the vehicle through the transparent region. Moreover, the sense of immersion further increases by decreasing the transmittance of the entire transparent FPD or increasing the display size of the content video.

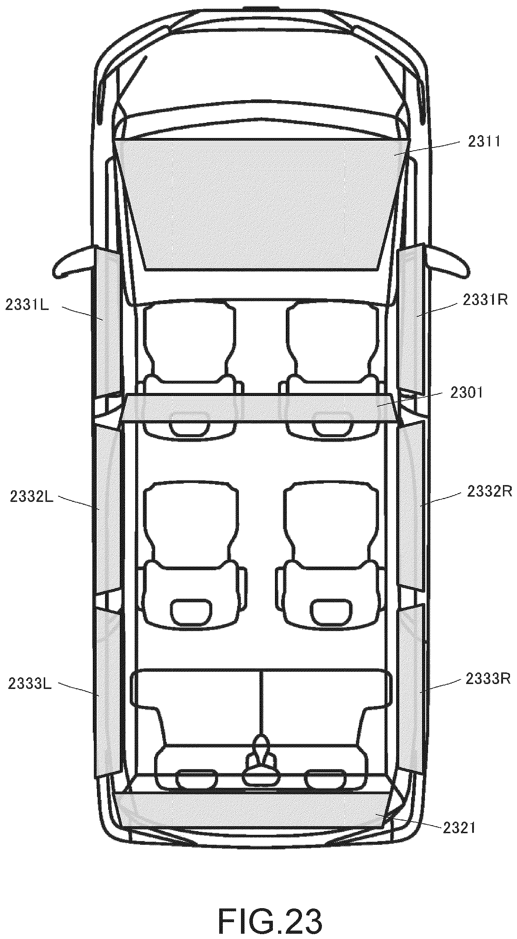

It should be noted that the above-mentioned display system for RSE is an example and the arrangement position of the transparent screen or the transparent FPD is not limited to that shown in Fig. 20. Fig. 23 shows a variation of the arrangement position of the transmittance variable region such as the transparent screen or the transparent FPD in the vehicle-mounted display system, taking a 3-row seat car as an example.

In Fig. 23, the reference numeral 2301 indicates the arrangement position of the transparent screen for RSE or the transparent FPD at the back of the first row seat (or in the front of the second row seat). The reference numeral 2311 indicates an example in which a front window (wind shield) is set as the arrangement position of the transparent screen or the transparent FPD. The reference numeral 2321 indicates an example in which the rear window is set as the arrangement position of the transparent screen or the transparent FPD. For example, in a case where the seats of the second or third row have been turned backwards, it is sufficient to display a content video for the occupant sitting in the seat facing backwards on the rear window. The reference numerals 2331R, 2331R, and 2333R indicate an example in which the right side windows in the first, second, and third rows are set as arrangement positions of the transparent screen or the transparent FPD, respectively. Moreover, the reference numerals 2331L, 2331L, and 2333L indicate an example in which the left side windows in the first, second, and third rows are set as arrangement positions of the transparent screen or the transparent FPD, respectively.

It should be noted that in a case where the transparent screen or the transparent FPD is arranged on the car window such as the front window, the rear window, or the side window, it is possible to use it as a display and also use it as a smoked glass with adjustable transmittance.

In a case where a transparent screen is used as the transmittance variable region, it is necessary to mount a projector that projects a content video on the transparent screen in the vehicle together with it. The projector arrangement position for each arrangement position of the transparent screen in the vehicle in a case of the display system using the combination of the transparent screen and the projector will be described.

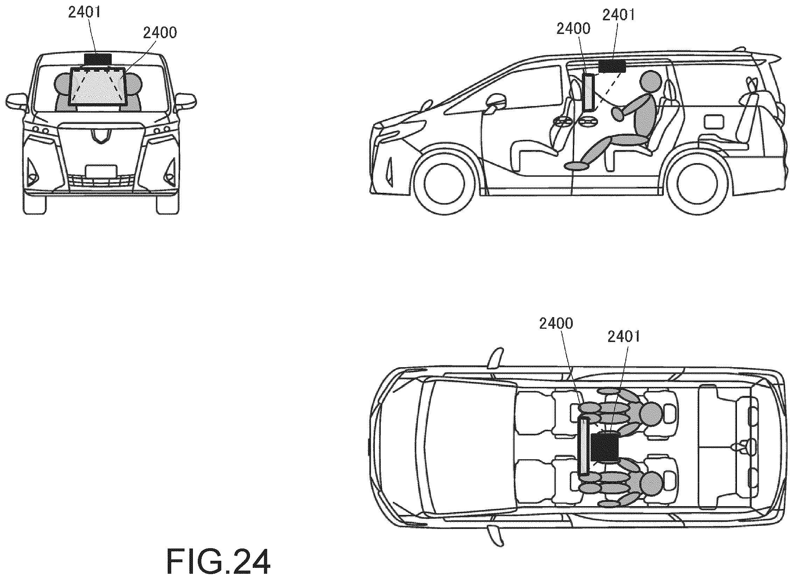

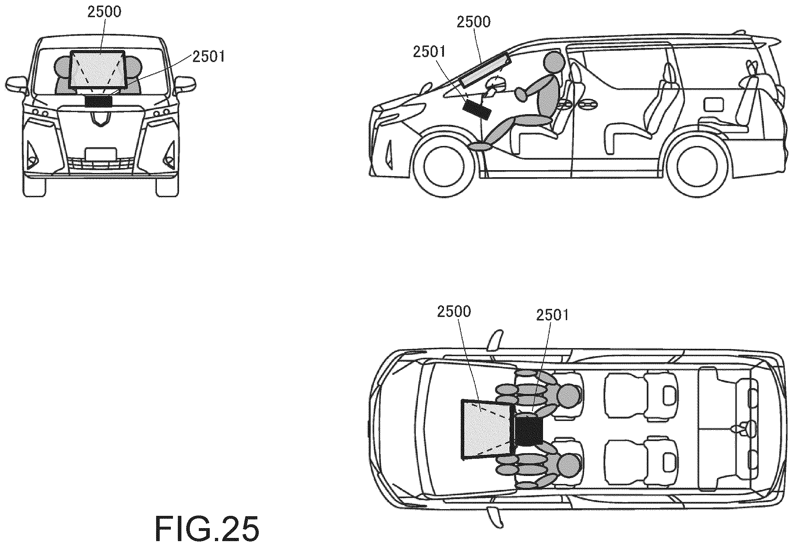

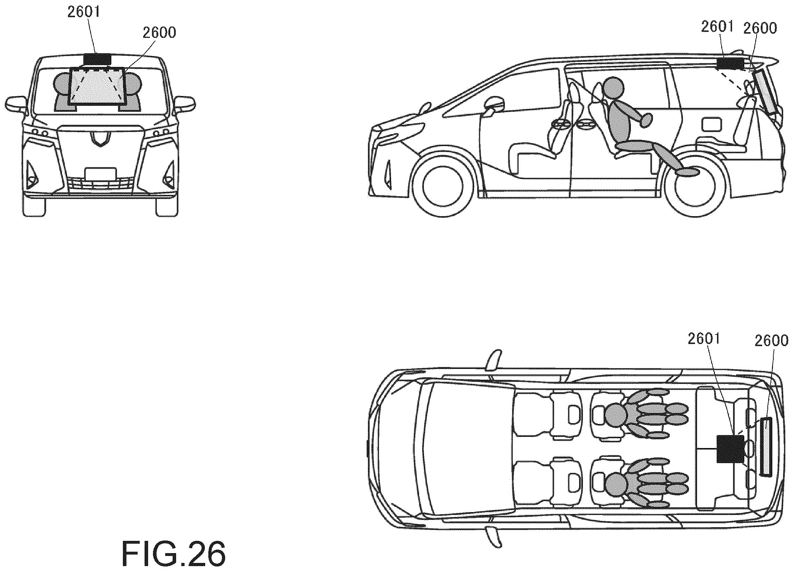

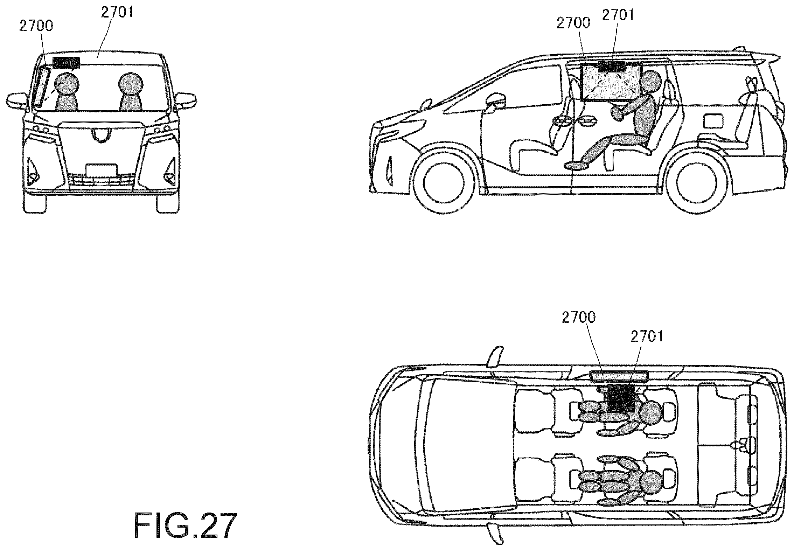

Fig. 24 illustrates an arrangement position of a projector 2401 in a case where a transparent screen 2400 for RSE is arranged at the back of the first row seat. Fig. 25 illustrates an arrangement position of a projector 2501 in a case where a transparent screen 2500 is arranged on the front window. Fig. 26 illustrates an arrangement position of a projector 2601 in a case where a transparent screen 2600 is arranged on the rear window. For example, in a case where the seats of the second or third row have been turned backwards, it is sufficient to display a content video for the occupant sitting in the seat facing backwards on the rear window. Fig. 27 illustrates an arrangement position of a projector 2701 in a case where a transparent screen 2700 is arranged on the side window.

In any example shown in Figs. 24 to 27, each projector 2401, 2501, 2601, 2701 is arranged on the bottom surface of the roof. Moreover, in any arrangement example, by arranging the projector in a position as close as possible to the transparent screen on which a content video is to be projected, it is possible to cause a video to be projected without being blocked by an object (e.g., an occupant) and to keep the maximum luminance high.

E-2. Reduction of Motion Sickness

For example, in a case where the display system for RSE has been mounted as shown in Fig. 20, the front field-of-view of the occupant sitting in the rear seat is occupied by a display (transparent screen or transparent FPD), and it is difficult to grasp the situation in the front of the vehicle. Therefore, there is a problem that it becomes difficult for the occupant to match the body to the motion of the vehicle, and the motion sickness easily occurs as a result.

In view of this, in the present disclosure, using the display system characteristics of displaying the content video in the transmittance variable region, in other words, of being capable of adjusting the transmittance of the video to be displayed, the motion sickness is reduced by controlling the transmittance of the transmittance variable region in accordance with a response level for motion sickness reduction considering the motion sickness index and the predicted displacement. Specifically, when the response level for motion sickness reduction has increased, the amount of field-of-view information in front of the vehicle through the entire display is increased by increasing the transmittance of the transparent screen or the transparent FPD. Accordingly, the visibility of the content video lowers, but it becomes easy to recognize the situation in the front of the vehicle, and it becomes possible to prevent development of the motion sickness by, for example, the occupant matching the body to the motion of the vehicle or to reduce the symptoms. Moreover, in accordance with the present disclosure, the motion sickness can also be reduced by changing the display position and the angle of the content video in accordance with the response level for motion sickness reduction to makes it easy for the occupant to match the posture to the displacement of the vehicle body.

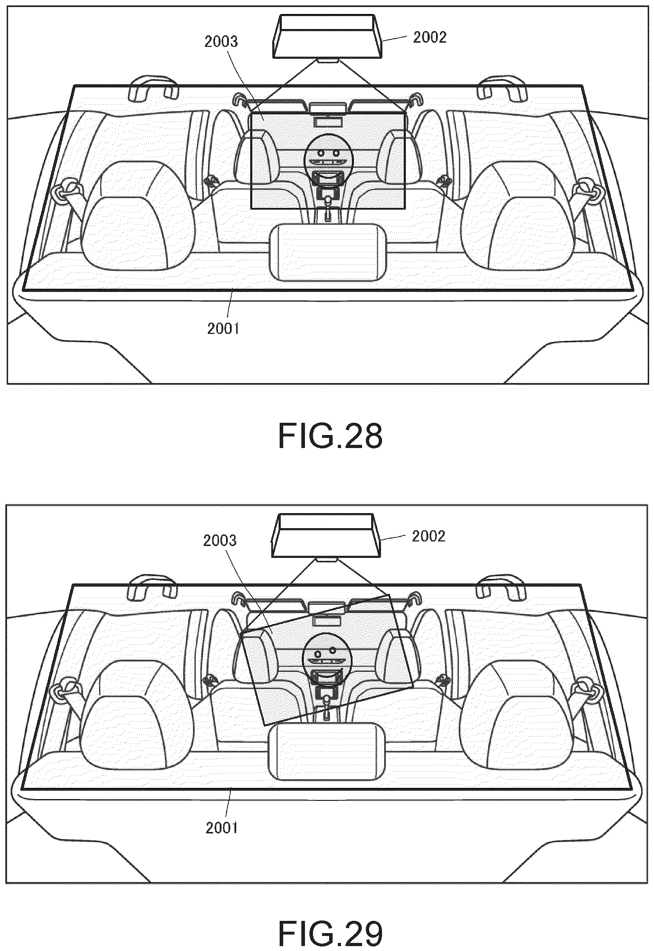

A display operation realized by the present disclosure will be described, exemplifying a display system for RSE, in other words, a display system mounting a display (e.g., transparent screen or transparent FPD) in the front of the vehicle as shown in Fig. 20. It is envisaged that for example in a case where there is a steep curve on the left-hand side in front of the vehicle, the occupant wishes to recognize the front so that the occupant can easily match the body to the displacement of the vehicle body for reducing the motion sickness. Figs. 28 to 35 each show an operation example of the display system for reducing the motion sickness in such a case.

In the example shown in Fig. 28, the content video 2003 displayed at the center of the transparent screen is made transparent. The occupant sitting in the rear seat has lower visibility of the content video 2003 as compared to the display example shown in Figs. 20 and 21, and the occupant can easily recognize the front of the vehicle and match the posture of the body to the displacement of the vehicle body, and therefore the motion sickness can be reduced.

In the example shown in Fig. 29, the content video 2003 displayed at the center of the transparent screen is made transparent and is rotated in a counter-clockwise direction in accordance with a sharp left turn of the vehicle body. Also in the display example shown in Fig. 29, the occupant sitting in the rear seat can easily recognize the front of the vehicle and match the posture of the body to the displacement of the vehicle body, and therefore the motion sickness can be reduced. Moreover, the content video 2003 is rotated in accordance with a tilt angle of the posture of the body which has been made to follow the displacement of the vehicle body during the sharp left turn, and therefore the visibility of the content video 2003 is further improved as compared to the display example shown in Fig. 28.

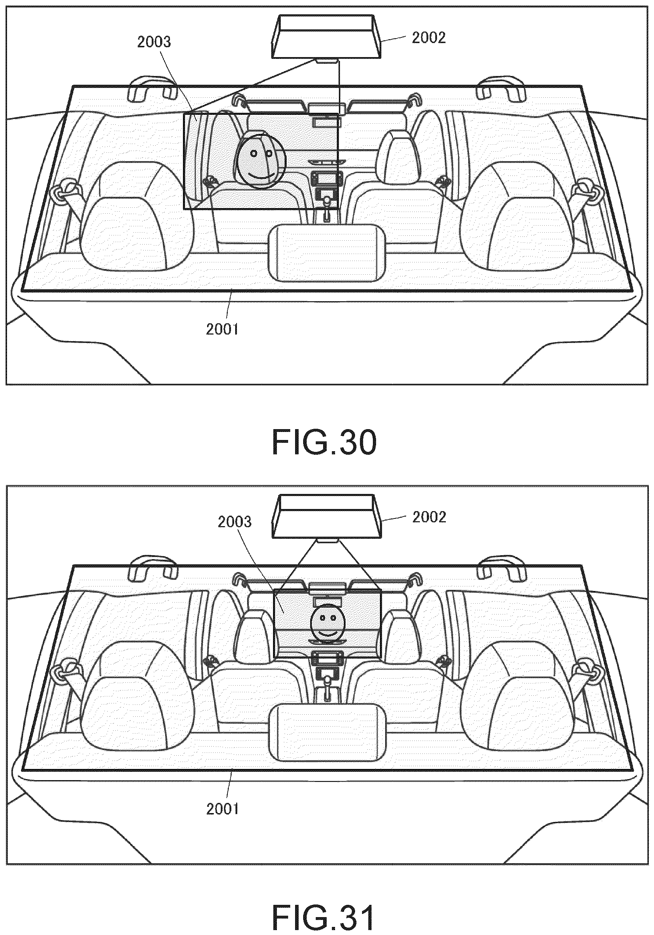

In the example shown in Fig. 30, the content video 2003 displayed at the center of the transparent screen is made transparent and is moved in accordance with a sharp left turn of the vehicle body. Also in the display example shown in Fig. 30, the occupant sitting in the rear seat can easily recognize the front of the vehicle and match the posture of the body to the displacement of the vehicle body, and therefore the motion sickness can be reduced. Since the display position of the content video 2003 is moved leftwards from the center of the transparent screen, the vehicle front field-of-view is further improved, and the occupant can more easily recognize the displacement of the vehicle body. Moreover, the content video 2003 is moved leftwards to be adapted to the position of the body tilted leftwards for withstanding the centrifugal force applied during the sharp left turn, and therefore the visibility of the content video 2003 is further improved as compared to the display example shown in Fig. 28.

In the example shown in Fig. 31, the content video 2003 displayed at the center of the transparent screen is made transparent and reduced in size. Also in the display example shown in Fig. 31, the occupant sitting in the rear seat can easily recognize the front of the vehicle and match the posture of the body to the displacement of the vehicle body, and therefore the motion sickness can be reduced. Due to the size reduction of the content video 2003, the visibility of the content video 2003 further lowers, but the vehicle front field-of-view is further improved, and the occupant can more easily recognize the displacement of the vehicle body.

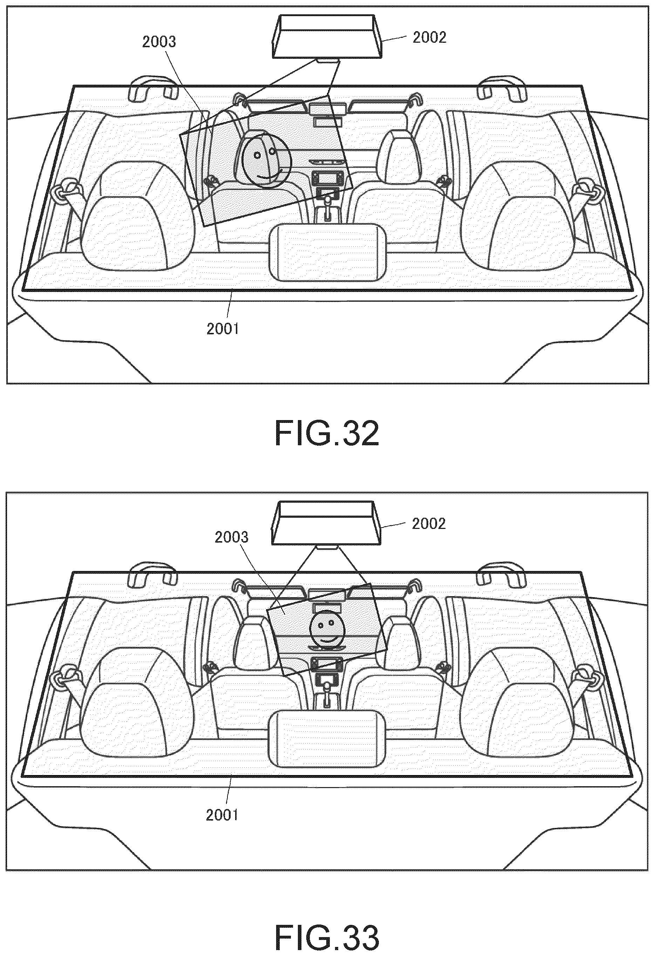

In the example shown in Fig. 32, the content video 2003 displayed at the center of the transparent screen is made transparent and is rotated in the counter-clockwise direction and moved leftwards in accordance with a sharp left turn of the vehicle body. Also in the display example shown in Fig. 32, the occupant sitting in the rear seat can easily recognize the front of the vehicle and match the posture of the body to the displacement of the vehicle body, and therefore the motion sickness can be reduced. Since the display position of the content video 2003 is moved leftwards from the center of the transparent screen, the vehicle front field-of-view is further improved, and the occupant can more easily recognize the displacement of the vehicle body. Moreover, the content video 2003 is rotated in accordance with a tilt angle of the posture of the body which has been made to follow the displacement of the vehicle body during the sharp left turn, and therefore the visibility of the content video 2003 is further improved as compared to the display example shown in Fig. 28.

In the example shown in Fig. 33, the content video 2003 displayed at the center of the transparent screen is made transparent and is rotated in the counter-clockwise direction and reduced in size in accordance with a sharp left turn of the vehicle body. Also in the display example shown in Fig. 33, the occupant sitting in the rear seat can easily recognize the front of the vehicle and match the posture of the body to the displacement of the vehicle body, and therefore the motion sickness can be reduced. Moreover, the content video 2003 is rotated in accordance with a tilt angle of the posture of the body which has been made to follow the displacement of the vehicle body during the sharp left turn, and therefore the visibility of the content video 2003 is further improved as compared to the display example shown in Fig. 28. Due to the size reduction of the content video 2003, the visibility of the content video 2003 further lowers, but the vehicle front field-of-view is further improved, and the occupant can more easily recognize the displacement of the vehicle body.

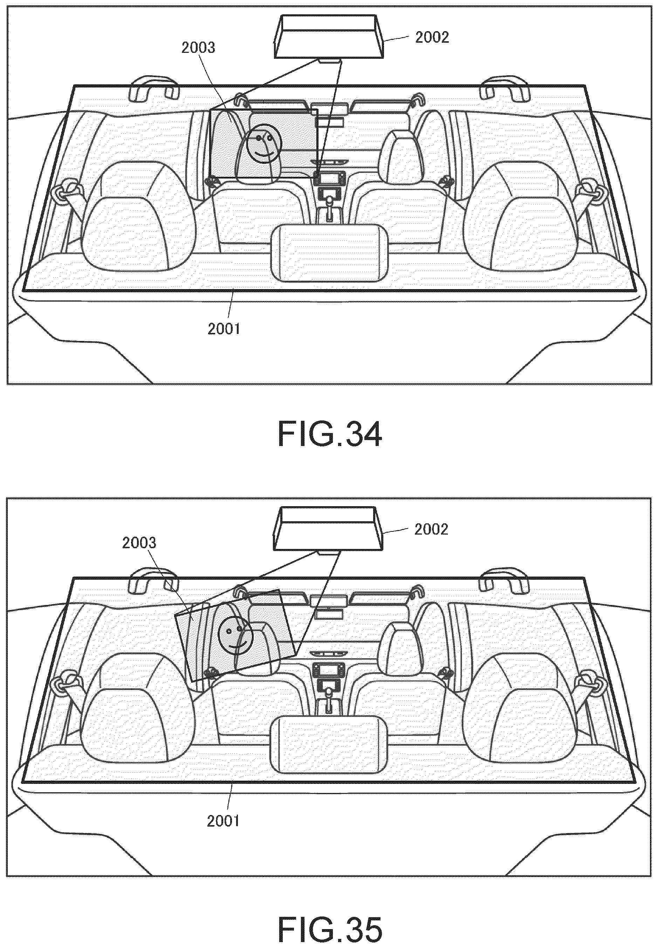

In the example shown in Fig. 34, the content video 2003 displayed at the center of the transparent screen is made transparent and is moved leftwards and reduced in size in accordance with a sharp left turn of the vehicle body. Also in the display example shown in Fig. 34, the occupant sitting in the rear seat can easily recognize the front of the vehicle and match the posture of the body to the displacement of the vehicle body, and therefore the motion sickness can be reduced. Since the display position of the content video 2003 is moved leftwards from the center of the transparent screen, the vehicle front field-of-view is further improved, and the occupant can more easily recognize the displacement of the vehicle body. Due to the size reduction of the content video 2003, the visibility of the content video 2003 further lowers, but the vehicle front field-of-view is further improved, and the occupant can more easily recognize the displacement of the vehicle body. Moreover, the visibility of the content video 2003 is improved by an amount corresponding to the left movement of the content video 2003 to be adapted to the position of the body tilted leftwards for withstanding the centrifugal force applied during the sharp left turn.

In the example shown in Fig. 35, the content video 2003 displayed at the center of the transparent screen is made transparent and is rotated in the counter-clockwise direction and moved leftwards and reduced in size in accordance with a sharp left turn of the vehicle body. Also in the display example shown in Fig. 35, the occupant sitting in the rear seat can easily recognize the front of the vehicle and match the posture of the body to the displacement of the vehicle body, and therefore the motion sickness can be reduced. Moreover, the visibility of the content video 2003 is improved by an amount corresponding to the rotation and movement of the content video 2003 according to the position and the posture of the body tilted and moved leftwards to follow the displacement of the vehicle body during the sharp left turn. Due to the size reduction of the content video 2003, the visibility of the content video 2003 further lowers, but the vehicle front field-of-view is further improved, and the occupant can more easily recognize the displacement of the vehicle body.

Fig. 29, Fig. 30, and Figs. 32 to 35 show the examples in which the content video made transparent on the display (e.g., transparent screen or transparent FPD) is rotated leftwards and moved leftwards when the vehicle makes a sharp left turn. Although the illustration is omitted, the effect of reducing the motion sickness of the occupant can also be obtained in such a manner that the content video made transparent on the display is rotated rightwards and moved rightwards when the vehicle makes a sharp right curve.

Moreover, the effect of reducing the motion sickness of the occupant can also be obtained in such a manner that the content video made transparent is moved upwards and downwards, not leftwards and rightwards, when the vehicle passes by a steep slope, not makes a steep curve. For example, when the vehicle goes up a steep ascent, the content video only needs to be moved upwards in accordance with upward movement of the line of sight of the occupant. In contrast, the vehicle goes down a steep descent, the content video only needs to be moved downwards in accordance with downward movement of the line of sight of the occupant.

The response processing unit 105 controls the transmittance of the transmittance variable region of the vehicle-mounted display system (or the content video displayed in the transmittance variable region) in accordance with a result of the motion sickness of the occupant which is calculated by the motion sickness computing unit 104, such that the motion sickness of the occupant can be reduced. Moreover, the response processing unit 105 changes the display size, the tilt, and the display position of the content video in addition to the transmittance of the transmittance variable region, such that the motion sickness of the occupant can be reduced while keeping the visibility of the content video.

F. Summary

Last of all, effects provided by the present disclosure are summarized. The following effects (1) and (2) are obtained in a vehicle such as the car to which the present disclosure is applied.

(1) In accordance with the present disclosure, the accumulation factor of motion sickness is further considered in addition to the accumulation factor of the motion sickness, and therefore more accurate motion sickness prediction corresponding to how the motion sickness appears in accordance with the traveling time of the vehicle, how to travel, and the like can be achieved.

(2) In accordance with the present disclosure, the head motion of the occupant is calculated by simulation on the basis of the motion of the vehicle when predicting the motion sickness on the basis of the head motion of the occupant. Therefore, it is unnecessary to directly detect the head motion that is difficult in measurement, and it is possible to acquire head motion data and perform the motion sickness prediction by detecting only vehicle acceleration that can be easily measured by the in-vehicle sensors.

Industrial Applicability

Hereinabove, the present disclosure has been described in detail with reference to specific embodiments. However, it is obvious that those skilled in the art may make modifications or substitutions to the embodiments without departing from the gist of the present disclosure.

Although this description has focused on the embodiments in which the present disclosure is applied to car sickness, the gist of the present disclosure is not limited thereto. The present disclosure can also be applied to motion sickness caused by motion of a vehicle (movable object) when an occupant boards various general vehicles (movable objects), such as seasickness.

In short, the present disclosure has been described by way of example and should not be construed as limiting the contents of the present disclosure. The scope of claims should be considered to determine the gist of the present disclosure.

It should be noted that the present disclosure can also take configurations as follows.

(1) An information processing apparatus, including:

a motion sickness accumulation computing unit that calculates an accumulation factor of motion sickness of an occupant in a movable object;
a motion sickness recovery computing unit that calculates a recovery factor of the motion sickness of the occupant; and
a motion sickness computing unit that combines the accumulation factor and the recovery factor to calculate a motion sickness index of the occupant.

(2) The information processing apparatus according to (1), in which
the motion sickness accumulation computing unit calculates the accumulation factor on the basis of a head motion of the occupant and a traveling time of the movable object.
(3) The information processing apparatus according to (2), in which
the motion sickness accumulation computing unit computes the accumulation factor by using a head motion of the occupant which is predicted by head motion simulation computing based on acceleration of the movable object and body information of the occupant.
(3-1) The information processing apparatus according to (3), in which
the body information of the occupant includes a sitting height and a weight of the occupant.
(3-2) The information processing apparatus according to (3), in which
the head simulation computing is dynamics calculation of approximating the movable object and the occupant as mechanical elements including a spring, a mass, and a damper and using the acceleration of the movable object and the body information of the occupant.
(4) The information processing apparatus according to any one of (1) to (3), in which
the motion sickness accumulation factor computing unit computes the accumulation factor on the basis of a motion sickness dose value (MSDV) defined by ISO 2631.
(5) The information processing apparatus according to any one of (1) to (4), in which
the motion sickness recovery factor computing unit computes the recovery factor on the basis of a traveling time of the movable object and body information of the occupant.
(6) The information processing apparatus according to (5), in which
the motion sickness recovery factor computing unit computes the recovery factor also in consideration of at least one of a position of the occupant in the movable object or a posture of the occupant.
(7) The information processing apparatus according to (6), in which
the motion sickness recovery factor computing unit computes the recovery factor in consideration of at least one of a motion of the movable object according to the position of the occupant or field-of-view information according to the posture of the occupant.
(8) The information processing apparatus according to any one of (5) to (7), in which
the motion sickness recovery factor computing unit computes the recovery factor also in consideration of how the movable object is traveling.
(8-1) The information processing apparatus according to (8), in which
information regarding how the movable object is traveling is acquired on the basis of the head motion of the occupant or positional information of the movable object.
(8-2) The information processing apparatus according to (8), in which
how the movable object is traveling includes at least one of which one the movable object is performing, manual driving or automated driving, a traveling speed in automated driving of the movable object, or where the movable object is traveling, in a city or on a highway.
(9) The information processing apparatus according to any one of (1) to (8), in which
the motion sickness computing unit calculates the motion sickness index on the basis of an computing result for a predetermined time or longer.
(10) The information processing apparatus according to (9), in which
the predetermined time is a time in which correlation between a motion sickness susceptibility questionnaire (MSSQ) and a simulator sickness questionnaire (SSQ) as proneness to the motion sickness is sufficiently high.
(11) The information processing apparatus according to any one of (1) to (10), further including
a processing unit that performs processing according to the motion sickness index of the occupant in the movable object.
(12) The information processing apparatus according to (11), in which
the processing unit performs, when the motion sickness of the occupant is predicted, at least one of

information presentation to instruct limitation of at least one of a traveling speed or a route change in manual driving of the movable object (or driving operation for reducing the simulator sickness questionnaire of the occupant),
driving control associated with limitation of at least one of a traveling speed or a route change in automated driving of the movable object (or linked to reduction of the simulator sickness questionnaire of the occupant), or
operation control of a display system mounted on the movable object.

(13) The information processing apparatus according to (12), in which

the processing unit adjusts, in accordance with the motion sickness index of the occupant, transmittance of at least a partial transmittance variable region of the display system that displays a video in the transmittance variable region.

(13-1) The system according to (13), in which

the display system includes a screen with adjustable transmittance, and
the processing unit performs, in accordance with the motion sickness index of the occupant, at least one adjustment of adjustment of transmittance of the screen, or a size, a display position, or a contrast of a content video to be displayed on the screen.

(14) An information processing method, including:

a motion sickness accumulation computing step of calculating an accumulation factor of motion sickness of an occupant in a movable object;
a motion sickness recovery computing step of calculating a recovery factor of the motion sickness of the occupant; and
a motion sickness computing step of combining the accumulation factor and the recovery factor to calculate a motion sickness index of the occupant.

(15) A system, including:

a motion sickness accumulation computing unit that calculates an accumulation factor of motion sickness of an occupant in a movable object;
a motion sickness recovery computing unit that calculates a recovery factor of the motion sickness of the occupant; and
a motion sickness computing unit that combines the accumulation factor and the recovery factor to calculate a motion sickness index of the occupant;
a display system that is mounted on the movable object and displays a video to be viewed by the occupant; and
a processing unit that performs processing according to the motion sickness index of the occupant on the display system.

(15-1) The system according to (14), in which

the display system includes a screen with adjustable transmittance, and
the processing unit performs, in accordance with the motion sickness index of the occupant, at least one adjustment of adjustment of the transmittance of the screen or a size, a display position, or a contrast of the content video to be displayed on the screen.

Reference Signs List

| | |
|---|---|
| 100 | information processing system |
| 101 | head motion computing unit |
| 102 | motion sickness accumulation factor computing unit |
| 103 | motion sickness recovery factor computing unit |
| 104 | motion sickness computing unit |
| 105 | response processing unit |
| 301 | filter processing unit |
| 302 | first human body transfer function calculation unit |
| 501 | transfer function calculation unit |
| 502 | integral calculation unit |
| 701 | occupant attribute calculation unit |
| 702 | second human body transfer function calculation unit |
| 1100 | information processing system |
| 1101 | head motion computing unit |
| 1102 | motion sickness accumulation factor computing unit |
| 1103 | motion sickness recovery factor computing unit |
| 1104 | motion sickness computing unit |

1105    response processing unit

**Claims**

1.  An information processing apparatus, comprising:

    a motion sickness accumulation computing unit that calculates an accumulation factor of motion sickness of an occupant in a movable object;
    a motion sickness recovery computing unit that calculates a recovery factor of the motion sickness of the occupant; and
    a motion sickness computing unit that combines the accumulation factor and the recovery factor to calculate a motion sickness index of the occupant.

2.  The information processing apparatus according to claim 1, wherein
    the motion sickness accumulation computing unit calculates the accumulation factor on a basis of a head motion of the occupant and a traveling time of the movable object.

3.  The information processing apparatus according to claim 2, wherein
    the motion sickness accumulation computing unit computes the accumulation factor by using a head motion of the occupant which is predicted by head motion simulation computing based on acceleration of the movable object and body information of the occupant.

4.  The information processing apparatus according to claim 1, wherein
    the motion sickness accumulation factor computing unit computes the accumulation factor on a basis of a motion sickness dose value (MSDV) defined by ISO 2631.

5.  The information processing apparatus according to claim 1, wherein
    the motion sickness recovery factor computing unit computes the recovery factor on a basis of a traveling time of the movable object and body information of the occupant.

6.  The information processing apparatus according to claim 5, wherein
    the motion sickness recovery factor computing unit computes the recovery factor also in consideration of at least one of a position of the occupant in the movable object or a posture of the occupant.

7.  The information processing apparatus according to claim 6, wherein
    the motion sickness recovery factor computing unit computes the recovery factor in consideration of at least one of a motion of the movable object according to the position of the occupant or field-of-view information according to the posture of the occupant.

8.  The information processing apparatus according to claim 5, wherein
    the motion sickness recovery factor computing unit computes the recovery factor also in consideration of how the movable object is traveling.

9.  The information processing apparatus according to claim 1, wherein
    the motion sickness computing unit calculates the motion sickness index on a basis of an computing result for a predetermined time or longer.

10. The information processing apparatus according to claim 9, wherein
    the predetermined time is a time in which correlation between a motion sickness susceptibility questionnaire (MSSQ) and a simulator sickness questionnaire (SSQ) as proneness to the motion sickness is sufficiently high.

11. The information processing apparatus according to claim 1, further comprising
    a processing unit that performs processing according to the motion sickness index of the occupant in the movable object.

12. The information processing apparatus according to claim 11, wherein
    the processing unit performs, when the motion sickness of the occupant is predicted, at least one of

information presentation to instruct limitation of at least one of a traveling speed or a route change in manual driving of the movable object (or driving operation for reducing the simulator sickness questionnaire of the occupant),

driving control associated with limitation of at least one of a traveling speed or a route change in automated driving of the movable object (or linked to reduction of the simulator sickness questionnaire of the occupant), or operation control of a display system mounted on the movable object.

13. The information processing apparatus according to claim 12, wherein

the processing unit adjusts, in accordance with the motion sickness index of the occupant, transmittance of at least a partial transmittance variable region of the display system that displays a video in the transmittance variable region.

14. An information processing method, comprising:

a motion sickness accumulation computing step of calculating an accumulation factor of motion sickness of an occupant in a movable object;

a motion sickness recovery computing step of calculating a recovery factor of the motion sickness of the occupant; and

a motion sickness computing step of combining the accumulation factor and the recovery factor to calculate a motion sickness index of the occupant.

15. A system, comprising:

a motion sickness accumulation computing unit that calculates an accumulation factor of motion sickness of an occupant in a movable object;

a motion sickness recovery computing unit that calculates a recovery factor of the motion sickness of the occupant; and

a motion sickness computing unit that combines the accumulation factor and the recovery factor to calculate a motion sickness index of the occupant;

a display system that is mounted on the movable object and displays a video to be viewed by the occupant; and

a processing unit that performs processing according to the motion sickness index of the occupant on the display system.

Information processing system 100

FIG.1

START

Detect occupant ⎯ S201

Acquire attribute data of occupant ⎯ S202

Detect traveling start of vehicle ⎯ S203

Acquire acceleration information of vehicle ⎯ S204

S205 ⎯ Calculate head motion

Read sitting height and weight information ⎯ S207

S206 ⎯ Calculate accumulation factor of motion sickness

Calculate recovery factor of motion sickness ⎯ S208

S209 ⎯ Integrate accumulation factor and recovery factor

Response processing ⎯ S210

END

FIG.2

| Vehicle acceleration | $a_x, a_y, a_z$ → | Filter processing unit 301 | → | First human body transfer function calculation unit 302 | $A_x, A_y, A_z$ → |

Occupant sitting height $h$ →

Occupant weight $w$ →

Head motion computing unit 101

# FIG.3

START

↓

Acquire acceleration information of vehicle — S401

↓

Filter processing — S402

↓

Acquire occupant weight information — S403

Calculate head motion — S405

Acquire occupant sitting height information — S404

↓

Calculate head acceleration — S406

↓

END

# FIG.4

Traveling time | t →

Head acceleration | $A_x, A_y, A_z$ →

Transfer function calculation unit 501 →

Integral calculation unit 502 → MSDV

↑

Motion sickness accumulation factor computing unit 102

# FIG.5

START

↓

S602 — Acquire head acceleration information

Acquire traveling time information — S601

↓

S603 — Transfer function processing ←

↓

S604 — Time integral calculation ←

↓

Calculate MSDV — S605

↓

END

# FIG.6

Traveling time $\xrightarrow{t}$ → Second human body transfer function calculation unit 702 →

Occupant sitting height $\xrightarrow{h}$

Occupant weight $\xrightarrow{w}$ → Occupant attribute calculation unit 701 →

Occupant position →

Occupant posture →

Motion sickness recovery factor computing unit 103

# FIG.7

START

Acquire occupant weight information — S801

Acquire occupant sitting height information — S802

Position and posture information reading processing — S803

Occupant attribute calculation processing — S804

Acquire traveling time information — S805

Transfer function calculation processing — S806

Recovery effect calculation processing — S807

END

# FIG.8

FIG.9

FIG.10

FIG.11

START

Detect occupant — S1201

Acquire attribute data of occupant — S1202

Detect traveling start of vehicle — S1203

Acquire acceleration information of vehicle — S1204

S1205 — Calculate head motion

Read sitting height and weight information — S1207

S1206 — Calculate accumulation factor of motion sickness

Calculate recovery factor of motion sickness — S1209

S1210 — Integrate accumulation factor and recovery factor

Read position and posture information — S1208

S1211 — Response processing

END

# FIG.12

$a(t)$ → $H(s)$ → $a_w(t)$

# FIG.13

$$\sqrt{MSDV_x^2 + MSDV_y^2}$$

● : Normal traveling          ● : Acceleration and deceleration traveling

FIG.14

FIG.15

FIG.16

FIG.17

FIG.18

(A)

(B)

FIG.19

FIG.20

FIG.21

# FIG.22

FIG.23

FIG.24

FIG.25

FIG.26

FIG.27

FIG.28

FIG.29

FIG.30

FIG.31

## FIG.32

## FIG.33

# FIG.34

# FIG.35

## INTERNATIONAL SEARCH REPORT

| International application No. |
| --- |
| **PCT/JP2023/017117** |

### A. CLASSIFICATION OF SUBJECT MATTER

***B60W 40/08***(2012.01)i; ***A61B 5/18***(2006.01)i; ***A61B 10/00***(2006.01)i; ***B60R 11/02***(2006.01)i; ***B60R 16/037***(2006.01)i; ***B60W 40/10***(2012.01)i; ***B60W 50/08***(2020.01)i; ***G08G 1/16***(2006.01)i
FI:  B60W40/08; A61B5/18; A61B10/00 W; B60R11/02 C; B60R16/037; B60W40/10; B60W50/08; G08G1/16 F

According to International Patent Classification (IPC) or to both national classification and IPC

### B. FIELDS SEARCHED

Minimum documentation searched (classification system followed by classification symbols)

B60W40/00-50/16; A61B5/18; A61B10/00; B60R11/02; B60R16/037; G08G1/00-1/16

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2006-34576 A (MATSUSHITA ELECTRIC IND CO LTD) 09 February 2006 (2006-02-09)<br>entire text | 1-15 |
| A | WO 2019/131116 A1 (SONY CORP) 04 July 2019 (2019-07-04)<br>entire text | 1-15 |
| A | JP 4882433 B2 (NISSAN MOTOR CO LTD) 22 February 2012 (2012-02-22)<br>entire text | 1-15 |
| A | JP 2012-59274 A (TOYOTA MOTOR CORP) 22 March 2012 (2012-03-22)<br>entire text | 1-15 |

☐ Further documents are listed in the continuation of Box C.  ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **04 July 2023** | **18 July 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/017117**

| Patent document cited in search report | | | Publication date (day/month/year) | Patent family member(s) | | | Publication date (day/month/year) |
|---|---|---|---|---|---|---|---|
| JP | 2006-34576 | A | 09 February 2006 | (Family: none) | | | |
| WO | 2019/131116 | A1 | 04 July 2019 | US entire text | 2020/0385025 | A1 | |
| JP | 4882433 | B2 | 22 February 2012 | JP entire text | 2007-236644 | A | |
| JP | 2012-59274 | A | 22 March 2012 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007236644 A
- JP 2005517568 A
- JP 2004286502 A